# EUROPEAN PATENT APPLICATION

(11) **EP 4 512 421 A1**
(43) Date of publication of application: **26.02.2025**
(21) Application number: 23803036.5
(22) Date of filing: 12.05.2023
(51) Int. Cl.: A61K 39/395, A61P 35/00

(54) **DRUG COMBINATION OF ANTI-TIM-3 ANTIBODY AND ANTI-PD-1 ANTIBODY**

(30) Priority: 13.05.2022 CN 202210523603
(71) Applicant: Nanjing Shunxin Pharmaceuticals Co., Ltd. of Chiatai Tianqing Pharmaceutical Group, Nanjing, Jiangsu 211100 (CN); Chia Tai Tianqing Pharmaceutical Group Co., Ltd., Lianyungang, Jiangsu 222062 (CN)
(72) Inventor: ZHAO, Qian, Nanjing, Jiangsu 211100 (CN); WANG, Xunqiang, Nanjing, Jiangsu 211100 (CN); ZHANG, Xiquan, Nanjing, Jiangsu 211100 (CN); YU, Ding, Nanjing, Jiangsu 211100 (CN); WANG, Liangliang, Nanjing, Jiangsu 211100 (CN)
(74) Representative: Grünecker Patent- und Rechtsanwälte PartG mbB
(86) International application number: PCT/CN2023/093865
(87) International publication number: WO 2023/217268

(57) **Abstract**

Provided is a drug combination of an anti-TIM-3 antibody and an anti-PD-1 antibody. The drug combination comprises an anti-TIM-3 antibody or an antigen-binding fragment thereof, and an anti-PD-1 antibody or an antigen-binding fragment thereof. Also provided is a kit for treating tumors, comprising the drug combination. In addition, further provided are an application of the drug combination in preparing a drug for treating tumors, and a method for treating tumors by using the drug combination.

## Description

The present disclosure claims priority to the Chinese Patent Application No. 202210523603.4 filed with China National Intellectual Property Administration on May 13, 2022 and entitled "DRUG COMBINATION OF ANTI-TIM-3 ANTIBODY AND ANTI-PD-1 ANTIBODY", the disclosure of which is incorporated herein by reference in its entirety.

### TECHNICAL FIELD

The present disclosure belongs to the field of biomedicine, and particularly relates to a pharmaceutical combination of an anti-TIM-3 antibody and an anti-PD-1 antibody.

### BACKGROUND

In recent years, the incidence of tumors has been on the rise, with malignant tumors showing poor treatment outcomes and high metastasis rates in advanced stages. At present, conventional treatment methods in the clinical use, such as radiotherapy, chemotherapy and surgery, can alleviate the pain and prolong the survival period, but they all have limitations. Therefore, immunotherapy, which kills tumor cells by inhibiting immune checkpoints, re-activating immune cells, and avoiding immune escape of tumor cells, has gradually become a hotspot in the field of tumor treatment.

T cell immunoglobulin and mucin domain-containing protein 3 (TIM-3), also known as hepatitis A virus cellular receptor 2 (HAVCR2), is a member of the immunomodulatory protein TIM family (the human TIM family includes TIM-1, 3, and 4). TIM-3 is selectively expressed on the surface of activated Th1 cells, and is also expressed on myeloid cells, DC cells, NK cells, and macrophages, as well as on various tumor cells. TIM-3 has a number of different ligands including galectin9, phosphatidylserine (PtdSer), high mobility group box 1 (HMGB1), and carcinoembryonic antigen cell adhesion molecule 1 (CEACAM1). TIM-3, as an immune checkpoint, has the physiological function of negatively regulating the immune function of an organism, avoiding the damage to the organism caused by overly strong immune function or autoimmune function. An increasing amount of evidence indicates that the TIM-3 protein and/or mRNA have up-regulated expression in various tumor tissues and tumor-associated immune cells, participate in tumor immune escape and immune response, and thus promote tumor development.

PD-1 (programmed death-1, programmed death receptor 1) is a key immune checkpoint receptor expressed by activated T lymphocytes and B lymphocytes and mediates immunosuppression, and its ligands include PD-L1 and PD-L2. An anti-PD-1 monoclonal antibody 14C12H1L1 was disclosed in Chinese Patent Application Publication No. CN106977602A, which can effectively block the binding of PD-1 to PD-L1, demonstrating relatively good anti-tumor activity.

The combination of an anti-TIM-3 antibody with an anti-PD-1 antibody can jointly block the signaling pathways of TIM-3 and PD-1, enhancing the anti-tumor effect.

### BRIEF SUMMARY

In one aspect, the present disclosure provides a pharmaceutical combination comprising an anti-TIM-3 antibody or an antigen-binding fragment thereof and an anti-PD-1 antibody or an antigen-binding fragment thereof.

In some embodiments, the pharmaceutical combination comprises the anti-TIM-3 antibody or the antigen-binding fragment thereof in a unit dose of 60-1800 mg and the anti-PD-1 antibody or the antigen-binding fragment thereof in a unit dose of 10-500 mg.

In some embodiments, the pharmaceutical combination comprises 100-1800 mg of the anti-TIM-3 antibody or the antigen-binding fragment thereof and 10-800 mg of the anti-PD-1 antibody or the antigen-binding fragment thereof.

In some embodiments, the pharmaceutical combination is suitable for administration within a single treatment cycle and comprises 100-1800 mg of the anti-TIM-3 antibody or the antigen-binding fragment thereof and 10-800 mg of the anti-PD-1 antibody or the antigen-binding fragment thereof.

In some embodiments, the pharmaceutical combination is for use in treating a tumor.

In another aspect, the present disclosure provides a pharmaceutical combination for use in treating a tumor, comprising an anti-TIM-3 antibody or an antigen-binding fragment thereof and an anti-PD-1 antibody or an antigen-binding fragment thereof.

In another aspect, the present disclosure further provides a method for treating a tumor in a subject, comprising administering to the subject a therapeutically effective amount of the pharmaceutical combination of the present disclosure. In addition, the present disclosure further provides use of the pharmaceutical combination of the present disclosure for preparing a medicament for use in treating a tumor. Alternatively, the present disclosure further provides use of the anti-TIM-3 antibody or the antigen-binding fragment thereof and the anti-PD-1 antibody or the antigen-binding fragment thereof for preparing a medicament for use in treating a tumor.

In some embodiments, the anti-TIM-3 antibody or the antigen-binding fragment thereof and the anti-PD-1 antibody or the antigen-binding fragment thereof may be packaged separately or packaged together. Moreover, the anti-TIM-3 antibody or the antigen-binding fragment thereof can be packaged in a single unit or multiple units, and the anti-PD-1 antibody or the antigen-binding fragment thereof can be packaged in a single unit or multiple units.

In some embodiments, the pharmaceutical combination is a fixed combination. In some embodiments, the fixed combination is present in the form of a solid pharmaceutical composition or a liquid pharmaceutical composition. In some embodiments, the pharmaceutical combination is a non-fixed combination. In some embodiments, the anti-TIM-3 antibody or the antigen-binding fragment thereof and the anti-PD-1 antibody or the antigen-binding fragment thereof in the non-fixed combination are each present in the form of a pharmaceutical composition.

In some embodiments, the anti-TIM-3 antibody or the antigen-binding fragment thereof and the anti-PD-1 antibody or the antigen-binding fragment thereof may be administered simultaneously, sequentially, and/or alternately.

In some embodiments, the anti-TIM-3 antibody or the antigen-binding fragment thereof is administered once every 1 week, every 2 weeks, every 3 weeks, or every 4 weeks. In some embodiments, the anti-TIM-3 antibody or the antigen-binding fragment thereof is administered at a dose of 100-1800 mg, 600-1800 mg, 600-1500 mg, or 600-1200 mg each time.

In some embodiments, the anti-PD-1 antibody or the antigen-binding fragment thereof is administered once every 1 week, every 2 weeks, every 3 weeks, or every 4 weeks. In some embodiments, the anti-PD-1 antibody or the antigen-binding fragment thereof is administered at a dose of 10-800 mg, 50-500 mg, or 100-200 mg each time. In another aspect, the present disclosure provides a kit for use in treating a tumor, comprising the pharmaceutical combination of the present disclosure. In some embodiments, the kit comprises the anti-TIM-3 antibody or the antigen-binding fragment thereof and the anti-PD-1 antibody or the antigen-binding fragment thereof.

### SUMMARY

The present disclosure provides a pharmaceutical combination comprising an anti-TIM-3 antibody or an antigen-binding fragment thereof and an anti-PD-1 antibody or an antigen-binding fragment thereof. In some embodiments, the pharmaceutical combination is for use in treating a tumor.

The present disclosure further provides use of the pharmaceutical combination of the present disclosure for preparing a medicament for use in treating a tumor. The present disclosure further provides use of the pharmaceutical combination of the present disclosure for treating a tumor. The present disclosure further provides a method for treating a tumor, comprising administering to a subject a therapeutically effective amount of the pharmaceutical combination of the present disclosure.

The present disclosure further provides a kit for use in treating a tumor, comprising the pharmaceutical combination of the present disclosure. In some embodiments, the kit further comprises an instruction for use of the pharmaceutical combination of the present disclosure in treating the tumor.

In addition, the present disclosure further provides an anti-TIM-3 antibody or an antigen-binding fragment thereof for use in treating a tumor. The present disclosure further provides use of the anti-TIM-3 antibody or the antigen-binding fragment thereof for preparing a medicament for use in treating a tumor. The present disclosure further provides use of the anti-TIM-3 antibody or the antigen-binding fragment thereof for treating a tumor. The present disclosure further provides a method for treating a tumor, comprising administering to a subject a therapeutically effective amount of the anti-TIM-3 antibody or the antigen-binding fragment thereof of the present disclosure.

In some embodiments, the tumor is a non-solid tumor. In some embodiments, the tumor is lymphoma. In some embodiments, the lymphoma is Hodgkin's lymphoma (HL) or non-Hodgkin's lymphoma (NHL).

### Pharmaceutical combination

In one aspect, the present disclosure provides a pharmaceutical combination comprising an anti-TIM-3 antibody or an antigen-binding fragment thereof and an anti-PD-1 antibody or an antigen-binding fragment thereof.

In some embodiments, the pharmaceutical combination is packaged in a kit further comprising an instruction for combined use of the anti-TIM-3 antibody or the antigen-binding fragment thereof and the anti-PD-1 antibody or the antigen-binding fragment thereof in treating a tumor. In other embodiments, the anti-TIM-3 antibody or the antigen-binding fragment thereof and the anti-PD-1 antibody or the antigen-binding fragment thereof in the pharmaceutical combination are packaged separately in their respective kits further comprising an instruction for combined use of the anti-TIM-3 antibody or the antigen-binding fragment thereof and the anti-PD-1 antibody or the antigen-binding fragment thereof in treating a tumor.

In some embodiments, the pharmaceutical combination is a fixed combination. In some embodiments, the fixed combination is present in the form of a solid pharmaceutical composition or a liquid pharmaceutical composition. In some embodiments, the anti-TIM-3 antibody or the antigen-binding fragment thereof and the anti-PD-1 antibody or the antigen-binding fragment thereof in the fixed combination are formulated in a single formulation. In some embodiments, the single formulation is present in the form of a liquid formulation or in the form of a solid formulation. In some specific embodiments, the single formulation is an injection. In some specific embodiments, the single formulation is a lyophilized formulation.

In some embodiments, the pharmaceutical combination is a non-fixed combination. In some embodiments, the anti-TIM-3 antibody or the antigen-binding fragment thereof and the anti-PD-1 antibody or the antigen-binding fragment thereof in the non-fixed combination are each present in the form of a pharmaceutical composition, and the pharmaceutical composition comprising the anti-TIM-3 antibody or the antigen-binding fragment thereof and the pharmaceutical composition comprising the anti-PD-1 antibody or the antigen-binding fragment thereof are present or not present in the same sachet. In some specific embodiments, in the non-fixed combination, the pharmaceutical composition comprising the anti-TIM-3 antibody or the antigen-binding fragment thereof is a liquid pharmaceutical composition. In some specific embodiments, in the non-fixed combination, the pharmaceutical composition comprising the anti-TIM-3 antibody or the antigen-binding fragment thereof is an injection. In some specific embodiments, in the non-fixed combination, the pharmaceutical composition comprising the anti-TIM-3 antibody or the antigen-binding fragment thereof is a solid pharmaceutical composition. In some specific embodiments, in the non-fixed combination, the pharmaceutical composition comprising the anti-TIM-3 antibody or the antigen-binding fragment thereof is a lyophilized formulation. In some specific embodiments, in the non-fixed combination, the pharmaceutical composition comprising the anti-PD-1 antibody or the antigen-binding fragment thereof is a liquid pharmaceutical composition. In some specific embodiments, in the non-fixed combination, the pharmaceutical composition comprising the anti-PD-1 antibody or the antigen-binding fragment thereof is an injection. In some specific embodiments, in the non-fixed combination, the pharmaceutical composition comprising the anti-PD-1 antibody or the antigen-binding fragment thereof is a solid pharmaceutical composition. In some specific embodiments, in the non-fixed combination, the pharmaceutical composition comprising the anti-PD-1 antibody or the antigen-binding fragment thereof is a lyophilized formulation.

It is also an object of the present disclosure to at least provide a pharmaceutical pack comprising separately packaged pharmaceutical compositions in separate containers, wherein a pharmaceutical composition comprising the anti-TIM-3 antibody or the antigen-binding fragment thereof is contained in a first container, and a pharmaceutical composition comprising the anti-PD-1 antibody or the antigen-binding fragment thereof is contained in a second container.

In some embodiments, a unit dose of the anti-TIM-3 antibody or the antigen-binding fragment thereof in the pharmaceutical combination is 60-1800 mg, 100-1600 mg, 120-1600 mg, 180-1200 mg, or 240-600 mg. In some embodiments, the unit dose of the anti-TIM-3 antibody or the antigen-binding fragment thereof in the pharmaceutical combination is about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, and/or about 1800 mg, or a range formed by any of the above values. In some embodiments, the unit dose of the anti-TIM-3 antibody or the antigen-binding fragment thereof in the pharmaceutical combination is about 240 mg, about 300 mg, about 360 mg, and/or about 600 mg. In some embodiments, the unit dose of the anti-TIM-3 antibody or the antigen-binding fragment thereof in the pharmaceutical combination is about 240 mg and/or about 600 mg.

In some embodiments, a unit dose of the anti-PD-1 antibody or the antigen-binding fragment thereof in the pharmaceutical combination is 10-500 mg, 50-500 mg, 50-200 mg, or 100-200 mg. In some embodiments, the unit dose of the anti-PD-1 antibody or the antigen-binding fragment thereof in the pharmaceutical combination is about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, and/or about 500 mg, or a range formed by any of the above values. In some embodiments, the unit dose of the anti-PD-1 antibody or the antigen-binding fragment thereof in the pharmaceutical combination is about 100 mg and/or about 200 mg. In some embodiments, the unit dose of the anti-PD-1 antibody or the antigen-binding fragment thereof in the pharmaceutical combination is about 100 mg. In some embodiments, the pharmaceutical combination comprises the anti-TIM-3 antibody or the antigen-binding fragment thereof in a unit dose of 60-1800 mg, 100-1600 mg, 120-1600 mg, 180-1200 mg, or 240-600 mg and the anti-PD-1 antibody or the antigen-binding fragment thereof in a unit dose of 10-500 mg, 50-500 mg, 50-200 mg, or 100-200 mg. In some embodiments, the pharmaceutical combination comprises the anti-TIM-3 antibody or the antigen-binding fragment thereof in a unit dose of about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, and/or about 1800 mg, or a range formed by any of the above values and the anti-PD-1 antibody or the antigen-binding fragment thereof in a unit dose of about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, and/or about 500 mg, or a range formed by any of the above values. In some embodiments, the pharmaceutical combination comprises the anti-TIM-3 antibody or the antigen-binding fragment thereof in a unit dose of about 240 mg, about 300 mg, about 360 mg, and/or about 600 mg and the anti-PD-1 antibody or the antigen-binding fragment thereof in a unit dose of about 100 mg and/or about 200 mg. In some embodiments, the pharmaceutical combination comprises the anti-TIM-3 antibody or the antigen-binding fragment thereof in a unit dose of about 240 mg and/or about 600 mg and the anti-PD-1 antibody or the antigen-binding fragment thereof in a unit dose of about 100 mg.

In some embodiments, the pharmaceutical combination comprises 100-1800 mg, 600-1800 mg, 600-1500 mg, or 600-1200 mg of the anti-TIM-3 antibody or the antigen-binding fragment thereof. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof. In some embodiments, the pharmaceutical combination comprises about 600 mg or about 1200 mg of the anti-TIM-3 antibody or the antigen-binding fragment thereof.

In some embodiments, the pharmaceutical combination comprises 10-800 mg, 50-500 mg, or 100-200 mg of the anti-PD-1 antibody or the antigen-binding fragment thereof. In some embodiments, the pharmaceutical combination comprises about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof. In some embodiments, the pharmaceutical combination comprises about 200 mg of the anti-PD-1 antibody or the antigen-binding fragment thereof.

In some embodiments, the pharmaceutical combination comprises 100-1800 mg, 600-1800 mg, 600-1500 mg, or 600-1200 mg of the anti-TIM-3 antibody or the antigen-binding fragment thereof and 10-800 mg, 50-500 mg, or 100-200 mg of the anti-PD-1 antibody or the antigen-binding fragment thereof. In some embodiments, the pharmaceutical combination comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof and about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof. In some embodiments, the pharmaceutical combination comprises about 600 mg or about 1200 mg of the anti-TIM-3 antibody or the antigen-binding fragment thereof and about 200 mg of the anti-PD-1 antibody or the antigen-binding fragment thereof.

In some embodiments, in the pharmaceutical combination, the content of the anti-TIM-3 antibody or the antigen-binding fragment thereof is a daily dose. In some embodiments, in the pharmaceutical combination, the content of the anti-TIM-3 antibody or the antigen-binding fragment thereof is a once-daily dose.

In some embodiments, in the pharmaceutical combination, the content of the anti-TIM-3 antibody or the antigen-binding fragment thereof is a flat dose.

In some embodiments, in the pharmaceutical combination, the content of the anti-TIM-3 antibody or the antigen-binding fragment thereof is a dose for one treatment cycle, each treatment cycle being 3 weeks (i.e., 21 days).

In some embodiments, in the pharmaceutical combination, the content of the anti-PD-1 antibody or the antigen-binding fragment thereof is a daily dose. In some embodiments, in the pharmaceutical combination, the content of the anti-PD-1 antibody or the antigen-binding fragment thereof is a once-daily dose.

In some embodiments, in the pharmaceutical combination, the content of the anti-PD-1 antibody or the antigen-binding fragment thereof is a flat dose.

In some embodiments, in the pharmaceutical combination, the content of the anti-PD-1 antibody or the antigen-binding fragment thereof is a dose for one treatment cycle, each treatment cycle being 3 weeks. In some embodiments, in the pharmaceutical combination, the content of the anti-PD-1 antibody or the antigen-binding fragment thereof is a dose for one treatment cycle, each treatment cycle being 2 weeks.

In some embodiments, the pharmaceutical combination is suitable for administration within a single treatment cycle and comprises 100-1800 mg, 600-1800 mg, 600-1500 mg, or 600-1200 mg of the anti-TIM-3 antibody or the antigen-binding fragment thereof. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof. In some embodiments, the pharmaceutical combination is suitable for administration within a single treatment cycle and comprises about 600 mg or about 1200 mg of the anti-TIM-3 antibody or the antigen-binding fragment thereof.

In some embodiments, the pharmaceutical combination is suitable for administration within a single treatment cycle and comprises 10-800 mg, 50-500 mg, or 100-200 mg of the anti-PD-1 antibody or the antigen-binding fragment thereof. In some embodiments, the pharmaceutical combination is suitable for administration within a single treatment cycle and comprises about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof. In some embodiments, the pharmaceutical combination is suitable for administration within a single treatment cycle and comprises about 200 mg of the anti-PD-1 antibody or the antigen-binding fragment thereof.

In some embodiments, the pharmaceutical combination is suitable for administration within a single treatment cycle and comprises the anti-TIM-3 antibody or the antigen-binding fragment thereof and the anti-PD-1 antibody or the antigen-binding fragment thereof. In some embodiments, the pharmaceutical combination is suitable for administration within a single treatment cycle and comprises 100-1800 mg, 600-1800 mg, 600-1500 mg, or 600-1200 mg of the anti-TIM-3 antibody or the antigen-binding fragment thereof and 10-800 mg, 50-500 mg, or 100-200 mg of the anti-PD-1 antibody or the antigen-binding fragment thereof. In some embodiments, the pharmaceutical combination is suitable for administration within a single treatment cycle and comprises about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof and about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof. In some embodiments, the pharmaceutical combination is suitable for administration within a single treatment cycle and comprises about 600 mg or about 1200 mg of the anti-TIM-3 antibody or the antigen-binding fragment thereof and about 200 mg of the anti-PD-1 antibody or the antigen-binding fragment thereof.

In some embodiments, the anti-TIM-3 antibody or the antigen-binding fragment thereof may be a pharmaceutical composition comprising the anti-TIM-3 antibody or the antigen-binding fragment thereof. The pharmaceutical composition comprising the anti-TIM-3 antibody or the antigen-binding fragment thereof is in a single dose or in multiple doses, preferably in multiple doses. The multiple doses consist of multiple single doses. The single dose is a pharmaceutical composition comprising about 240 mg, about 300 mg, about 360 mg, and/or about 600 mg of the anti-TIM-3 antibody or the antigen-binding fragment thereof. Preferably, the single dose is a pharmaceutical composition comprising about 240 mg and/or about 600 mg of the anti-TIM-3 antibody or the antigen-binding fragment thereof.

In some embodiments, the anti-PD-1 antibody or the antigen-binding fragment thereof may be a pharmaceutical composition comprising the anti-PD-1 antibody or the antigen-binding fragment thereof. The pharmaceutical composition comprising the anti-PD-1 antibody or the antigen-binding fragment thereof is in a single dose or in multiple doses, preferably in multiple doses. The multiple doses consist of multiple single doses. The single dose is a pharmaceutical composition comprising about 100 mg and/or about 200 mg of the anti-PD-1 antibody or the antigen-binding fragment thereof. Preferably, the single dose is a pharmaceutical composition comprising about 100 mg of the anti-PD-1 antibody or the antigen-binding fragment thereof.

In some embodiments, in the pharmaceutical combination, a mass ratio of the anti-TIM-3 antibody or the antigen-binding fragment thereof to the anti-PD-1 antibody or the antigen-binding fragment thereof is (0.1-180):1, (0.2-50):1, (0.2-9):1, (0.5-9):1, or (3-6):1, wherein the anti-TIM-3 antibody or the antigen-binding fragment thereof and the anti-PD-1 antibody or the antigen-binding fragment thereof may be packaged separately or packaged together. In some embodiments, the anti-TIM-3 antibody or the antigen-binding fragment thereof and the anti-PD-1 antibody or the antigen-binding fragment thereof are packaged separately, wherein the anti-TIM-3 antibody or the antigen-binding fragment thereof can be packaged in a single unit or multiple units, and the anti-PD-1 antibody or the antigen-binding fragment thereof can be packaged in a single unit or multiple units. In some embodiments, the anti-TIM-3 antibody or the antigen-binding fragment thereof and the anti-PD-1 antibody or the antigen-binding fragment thereof are packaged separately, wherein the anti-TIM-3 antibody or the antigen-binding fragment thereof can be packaged in a single unit or multiple aliquots (e.g., 2 aliquots, 3 aliquots, 4 aliquots, 5 aliquots, 6 aliquots, 7 aliquots, 8 aliquots, or more aliquots), and the anti-PD-1 antibody or the antigen-binding fragment thereof can be packaged in a single unit or multiple aliquots (e.g., 2 aliquots or other aliquots). In some embodiments, the anti-TIM-3 antibody or the antigen-binding fragment thereof and the anti-PD-1 antibody or the antigen-binding fragment thereof are packaged together.

In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1500 mg, or 600-1200 mg of the anti-TIM-3 antibody or the antigen-binding fragment thereof, and the pharmaceutical composition comprising the anti-PD-1 antibody or the antigen-binding fragment thereof is prepared as a single dose or multiple doses suitable for administering to a patient 10-800 mg, 50-500 mg, or 100-200 mg of the anti-PD-1 antibody or the antigen-binding fragment thereof. In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof, and the pharmaceutical composition comprising the anti-PD-1 antibody or the antigen-binding fragment thereof is prepared as a single dose or multiple doses suitable for administering to a patient about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof. In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient about 600 mg or about 1200 mg of the anti-TIM-3 antibody or the antigen-binding fragment thereof, and the pharmaceutical composition comprising the anti-PD-1 antibody or the antigen-binding fragment thereof is prepared as a single dose or multiple doses suitable for administering to a patient about 200 mg of the anti-PD-1 antibody or the antigen-binding fragment thereof.

In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient 100-1800 mg, 600-1800 mg, 600-1500 mg, or 600-1200 mg of the anti-TIM-3 antibody or the antigen-binding fragment thereof at the first administration, and the pharmaceutical composition comprising the anti-PD-1 antibody or the antigen-binding fragment thereof is prepared as a single dose or multiple doses suitable for administering to a patient 10-800 mg, 50-500 mg, or 100-200 mg of the anti-PD-1 antibody or the antigen-binding fragment thereof at the first administration. In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values of the anti-TIM-3 antibody or the antigen-binding fragment thereof at the first administration, and the pharmaceutical composition comprising the anti-PD-1 antibody or the antigen-binding fragment thereof is prepared as a single dose or multiple doses suitable for administering to a patient about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values of the anti-PD-1 antibody or the antigen-binding fragment thereof at the first administration. In some embodiments, the pharmaceutical combination comprises a pharmaceutical composition comprising the anti-TIM-3 antibody or the antigen-binding fragment thereof and a pharmaceutical composition comprising the anti-PD-1 antibody or the antigen-binding fragment thereof, wherein the pharmaceutical composition comprising the anti-TIM-3 antibody or the antigen-binding fragment thereof is prepared as a single dose or multiple doses suitable for administering to a patient about 600 mg or about 1200 mg of the anti-TIM-3 antibody or the antigen-binding fragment thereof at the first administration, and the pharmaceutical composition comprising the anti-PD-1 antibody or the antigen-binding fragment thereof is prepared as a single dose or multiple doses suitable for administering to a patient about 200 mg of the anti-PD-1 antibody or the antigen-binding fragment thereof at the first administration.

In another aspect, the present disclosure further provides use of the pharmaceutical combination comprising the anti-TIM-3 antibody or the antigen-binding fragment thereof and the anti-PD-1 antibody or the antigen-binding fragment thereof for preparing a medicament for use in treating a tumor. The present disclosure further provides use of the pharmaceutical combination comprising the anti-TIM-3 antibody or the antigen-binding fragment thereof and the anti-PD-1 antibody or the antigen-binding fragment thereof for treating a tumor. The present disclosure further provides a method for treating a tumor in a subject, comprising administering to the subject a therapeutically effective amount of the pharmaceutical combination comprising the anti-TIM-3 antibody or the antigen-binding fragment thereof and the anti-PD-1 antibody or the antigen-binding fragment thereof.

In another aspect, the present disclosure provides a kit for use in treating a tumor, wherein the kit comprises a pharmaceutical composition comprising the anti-TIM-3 antibody or the antigen-binding fragment thereof and a pharmaceutical composition comprising the anti-PD-1 antibody or the antigen-binding fragment thereof, and an instruction for combined use of the pharmaceutical composition comprising the anti-TIM-3 antibody or the antigen-binding fragment thereof and the pharmaceutical composition comprising the anti-PD-1 antibody or the antigen-binding fragment thereof in treating the tumor.

In another aspect, the present disclosure provides a kit for use in treating a tumor, wherein the kit comprises a single formulation comprising the anti-TIM-3 antibody or the antigen-binding fragment thereof and the anti-PD-1 antibody or the antigen-binding fragment thereof, and an instruction for use of the single formulation in treating the tumor.

In another aspect, the present disclosure further provides a pharmaceutical pack for use in treating a tumor, comprising separately packaged pharmaceutical compositions in separate containers, wherein a pharmaceutical composition comprising the anti-TIM-3 antibody or the antigen-binding fragment thereof is contained in a first container, and a pharmaceutical composition comprising the anti-PD-1 antibody or the antigen-binding fragment thereof is contained in a second container.

In another aspect, the present disclosure further provides a pharmaceutical pack for use in treating a tumor, comprising separately packaged pharmaceutical compositions in separate containers, wherein a single formulation comprising the anti-TIM-3 antibody or the antigen-binding fragment thereof and the anti-PD-1 antibody or the antigen-binding fragment thereof is contained in the container.

In some specific embodiments, in the kit or the pharmaceutical pack, the pharmaceutical composition comprising the anti-TIM-3 antibody or the antigen-binding fragment thereof is a liquid pharmaceutical composition or a solid pharmaceutical composition. In some specific embodiments, the pharmaceutical composition comprising the anti-TIM-3 antibody or the antigen-binding fragment thereof is an injection. In some specific embodiments, the pharmaceutical composition comprising the anti-TIM-3 antibody or the antigen-binding fragment thereof is a lyophilized formulation.

In some specific embodiments, in the kit or the pharmaceutical pack, the pharmaceutical composition comprising the anti-PD-1 antibody or the antigen-binding fragment thereof is a liquid pharmaceutical composition or a solid pharmaceutical composition. In some specific embodiments, the pharmaceutical composition comprising the anti-PD-1 antibody or the antigen-binding fragment thereof is an injection. In some specific embodiments, the pharmaceutical composition comprising the anti-PD-1 antibody or the antigen-binding fragment thereof is a lyophilized formulation.

In some specific embodiments, in the kit or the pharmaceutical pack, the single formulation comprising the anti-TIM-3 antibody or the antigen-binding fragment thereof and the anti-PD-1 antibody or the antigen-binding fragment thereof is a liquid formulation or a solid formulation. In some specific embodiments, the single formulation comprising the anti-TIM-3 antibody or the antigen-binding fragment thereof and the anti-PD-1 antibody or the antigen-binding fragment thereof is an injection. In some specific embodiments, the single formulation comprising the anti-TIM-3 antibody or the antigen-binding fragment thereof and the anti-PD-1 antibody or the antigen-binding fragment thereof is a lyophilized formulation.

In some embodiments, the tumor is a non-solid tumor. In some embodiments, the tumor is lymphoma. In some embodiments, the lymphoma is Hodgkin's lymphoma or non-Hodgkin's lymphoma.

In some embodiments, the Hodgkin's lymphoma is classical Hodgkin's lymphoma (cHL) or nodular lymphocyte-predominant Hodgkin's lymphoma.

In some embodiments, the non-Hodgkin's lymphoma is T-cell non-Hodgkin's lymphoma (T-NHL) or B-cell non-Hodgkin's lymphoma (B-NHL). In some embodiments, the non-Hodgkin's lymphoma is aggressive non-Hodgkin's lymphoma or indolent non-Hodgkin's lymphoma. In some embodiments, the non-Hodgkin's lymphoma is diffuse large B-cell lymphoma (DLBCL), follicular lymphoma (FL), peripheral T-cell lymphoma (PTCL), mantle cell lymphoma (MCL), Burkitt lymphoma (BL), lymphoblastic lymphoma (LBL), cutaneous T-cell lymphoma, cutaneous B-cell lymphoma, marginal zone lymphoma, and/or AIDS-related B-cell lymphoma.

### Administration/treatment regimen of pharmaceutical combination

In some embodiments, in the use or the method for the treatment of the present disclosure, the anti-TIM-3 antibody or the antigen-binding fragment thereof and the anti-PD-1 antibody or the antigen-binding fragment thereof may be administered simultaneously, sequentially, and/or alternately. In some embodiments, in the use or the method for the treatment of the present disclosure, the anti-TIM-3 antibody or the antigen-binding fragment thereof and the anti-PD-1 antibody or the antigen-binding fragment thereof are administered sequentially. In some embodiments, in the use or the method for the treatment of the present disclosure, the anti-TIM-3 antibody or the antigen-binding fragment thereof and the anti-PD-1 antibody or the antigen-binding fragment thereof are each present in the form of a pharmaceutical composition, and may be administered simultaneously, sequentially, and/or alternately. In some embodiments, in the use or the method for the treatment of the present disclosure, the anti-TIM-3 antibody or the antigen-binding fragment thereof and the anti-PD-1 antibody or the antigen-binding fragment thereof are each present in the form of a pharmaceutical composition, and are administered sequentially. In some embodiments, in the use or the method for the treatment of the present disclosure, the anti-PD-1 antibody or the antigen-binding fragment thereof is administered first, followed by the anti-TIM-3 antibody or the antigen-binding fragment thereof. In some embodiments, in the use or the method for the treatment of the present disclosure, the anti-TIM-3 antibody or the antigen-binding fragment thereof and the anti-PD-1 antibody or the antigen-binding fragment thereof are each present in the form of a pharmaceutical composition, and the pharmaceutical composition comprising the anti-PD-1 antibody or the antigen-binding fragment thereof is administered first, followed by the pharmaceutical composition comprising the anti-TIM-3 antibody or the antigen-binding fragment thereof. In some embodiments, in the use and the method of the present disclosure, the anti-TIM-3 antibody or the antigen-binding fragment thereof and the anti-PD-1 antibody or the antigen-binding fragment thereof are formulated in a single formulation, and are administered simultaneously.

In some embodiments, in the use or the method for the treatment of the present disclosure, the anti-TIM-3 antibody or the antigen-binding fragment thereof and the anti-PD-1 antibody or the antigen-binding fragment thereof are separately administered according to identical or different administration regimens. In some embodiments, in the use and the method of the present disclosure, the anti-TIM-3 antibody or the antigen-binding fragment thereof and the anti-PD-1 antibody or the antigen-binding fragment thereof are separately administered according to identical administration regimens. In some embodiments, in the use and the method of the present disclosure, the anti-TIM-3 antibody or the antigen-binding fragment thereof and the anti-PD-1 antibody or the antigen-binding fragment thereof are separately administered according to different administration regimens.

In some embodiments, in the use or the method for the treatment of the present disclosure, the anti-TIM-3 antibody or the antigen-binding fragment thereof is administered once every 1 week (q1w), every 2 weeks (q2w), every 3 weeks (q3w), or every 4 weeks (q4w). In one specific embodiment, the anti-TIM-3 antibody or the antigen-binding fragment thereof is administered once every 3 weeks. In one specific embodiment, the anti-TIM-3 antibody or the antigen-binding fragment thereof is administered once every 4 weeks. In some embodiments, the anti-TIM-3 antibody or the antigen-binding fragment thereof is administered at a dose of 100-1800 mg, 600-1800 mg, 600-1500 mg, or 600-1200 mg each time. In some embodiments, the anti-TIM-3 antibody or the antigen-binding fragment thereof is administered at a dose of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg, or a range formed by any of the above values each time. In some embodiments, the anti-TIM-3 antibody or the antigen-binding fragment thereof is administered at a dose of about 600 mg or about 1200 mg each time. In some embodiments, the anti-TIM-3 antibody or the antigen-binding fragment thereof is administered once every 1 week, every 2 weeks, every 3 weeks, or every 4 weeks at a dose of 100-1800 mg, 600-1800 mg, 600-1500 mg, or 600-1200 mg of the anti-TIM-3 antibody or the antigen-binding fragment thereof each time. In some embodiments, the anti-TIM-3 antibody or the antigen-binding fragment thereof is administered once every 1 week, every 2 weeks, every 3 weeks, or every 4 weeks at a dose of 600-1200 mg of the anti-TIM-3 antibody or the antigen-binding fragment thereof each time. In some embodiments, the anti-TIM-3 antibody or the antigen-binding fragment thereof is administered once every 1 week, every 2 weeks, every 3 weeks, or every 4 weeks at a dose of about 600 mg or about 1200 mg of the anti-TIM-3 antibody or the antigen-binding fragment thereof each time. In some embodiments, the anti-TIM-3 antibody or the antigen-binding fragment thereof is administered once every 3 weeks at a dose of 600-1200 mg of the anti-TIM-3 antibody or the antigen-binding fragment thereof each time. In some embodiments, the anti-TIM-3 antibody or the antigen-binding fragment thereof is administered once every 3 weeks at a dose of about 600 mg or about 1200 mg of the anti-TIM-3 antibody or the antigen-binding fragment thereof each time.

In some embodiments, in the use or the method for the treatment of the present disclosure, the anti-PD-1 antibody or the antigen-binding fragment thereof is administered once every 1 week (q1w), every 2 weeks (q2w), every 3 weeks (q3w), or every 4 weeks (q4w). In one specific embodiment, the anti-PD-1 antibody or the antigen-binding fragment thereof is administered once every 2 weeks. In one specific embodiment, the anti-PD-1 antibody or the antigen-binding fragment thereof is administered once every 3 weeks. In some embodiments, the anti-PD-1 antibody or the antigen-binding fragment thereof is administered at a dose of 10-800 mg, 50-500 mg, or 100-200 mg each time. In some embodiments, the anti-PD-1 antibody or the antigen-binding fragment thereof is administered at a dose of about 10 mg, about 50 mg, about 100 mg, about 120 mg, about 140 mg, about 160 mg, about 180 mg, about 200 mg, about 220 mg, about 240 mg, about 260 mg, about 280 mg, about 300 mg, about 350 mg, about 400 mg, about 450 mg, about 500 mg, about 550 mg, about 600 mg, about 650 mg, about 700 mg, about 750 mg, or about 800 mg, or a range formed by any of the above values each time. In some embodiments, the anti-PD-1 antibody or the antigen-binding fragment thereof is administered at a dose of about 200 mg each time. In some embodiments, the anti-PD-1 antibody or the antigen-binding fragment thereof is administered once every 1 week, every 2 weeks, every 3 weeks, or every 4 weeks at a dose of 10-800 mg, 50-500 mg, or 100-200 mg of the anti-PD-1 antibody or the antigen-binding fragment thereof each time. In some embodiments, the anti-PD-1 antibody or the antigen-binding fragment thereof is administered once every 1 week, every 2 weeks, every 3 weeks, or every 4 weeks at a dose of 100-200 mg of the anti-PD-1 antibody or the antigen-binding fragment thereof each time. In some embodiments, the anti-PD-1 antibody or the antigen-binding fragment thereof is administered once every 1 week, every 2 weeks, every 3 weeks, or every 4 weeks at a dose of about 200 mg of the anti-PD-1 antibody or the antigen-binding fragment thereof each time. In some embodiments, the anti-PD-1 antibody or the antigen-binding fragment thereof is administered once every 2 weeks at a dose of about 200 mg of the anti-PD-1 antibody or the antigen-binding fragment thereof each time. In some embodiments, the anti-PD-1 antibody or the antigen-binding fragment thereof is administered once every 3 weeks at a dose of 100-200 mg of the anti-PD-1 antibody or the antigen-binding fragment thereof each time. In some embodiments, the anti-PD-1 antibody or the antigen-binding fragment thereof is administered once every 3 weeks at a dose of about 200 mg of the anti-PD-1 antibody or the antigen-binding fragment thereof each time.

In some embodiments, in the use and the method of the present disclosure, the anti-TIM-3 antibody or the antigen-binding fragment thereof and the anti-PD-1 antibody or the antigen-binding fragment thereof have identical or different treatment cycles. In some specific embodiments, the anti-TIM-3 antibody or the antigen-binding fragment thereof and the anti-PD-1 antibody or the antigen-binding fragment thereof have identical treatment cycles, for example, one treatment cycle is every 1 week, every 2 weeks, every 3 weeks, or every 4 weeks. In some specific embodiments, the anti-TIM-3 antibody or the antigen-binding fragment thereof and the anti-PD-1 antibody or the antigen-binding fragment thereof have identical treatment cycles, for example, one treatment cycle is every 3 weeks.

In some embodiments, in the use or the method for the treatment of the present disclosure, one treatment cycle is every 3 weeks, and the anti-TIM-3 antibody or the antigen-binding fragment thereof and the anti-PD-1 antibody or the antigen-binding fragment thereof are administered during each treatment cycle. In some embodiments, in the use and the method of the present disclosure, one treatment cycle is every 3 weeks, and the anti-TIM-3 antibody or the antigen-binding fragment thereof and the anti-PD-1 antibody or the antigen-binding fragment thereof are administered once separately during each treatment cycle. In some embodiments, in the use and the method of the present disclosure, one treatment cycle is every 3 weeks, and the anti-TIM-3 antibody or the antigen-binding fragment thereof is administered on day 1 of each treatment cycle and the anti-PD-1 antibody or the antigen-binding fragment thereof is administered on day 1 of each treatment cycle. In some embodiments, in the use and the method of the present disclosure, one treatment cycle is every 3 weeks, and the anti-TIM-3 antibody or the antigen-binding fragment thereof is administered once on day 1 of each treatment cycle and the anti-PD-1 antibody or the antigen-binding fragment thereof is administered once on day 1 of each treatment cycle. In some specific embodiments, in the use and the method of the present disclosure, one treatment cycle is every 3 weeks, and 600-1200 mg of the anti-TIM-3 antibody or the antigen-binding fragment thereof is administered during each treatment cycle and 100-200 mg of the anti-PD-1 antibody or the antigen-binding fragment thereof is administered during each treatment cycle. In some specific embodiments, in the use and the method of the present disclosure, one treatment cycle is every 3 weeks, and about 600 mg or about 1200 mg of the anti-TIM-3 antibody or the antigen-binding fragment thereof is administered during each treatment cycle and about 200 mg of the anti-PD-1 antibody or the antigen-binding fragment thereof is administered during each treatment cycle. In some specific embodiments, in the use and the method of the present disclosure, one treatment cycle is every 3 weeks, and about 600 mg or about 1200 mg of the anti-TIM-3 antibody or the antigen-binding fragment thereof is administered on day 1 of each treatment cycle and about 200 mg of the anti-PD-1 antibody or the antigen-binding fragment thereof is administered on day 1 of each treatment cycle.

In some embodiments, in the use and the method of the present disclosure, one treatment cycle is every 3 weeks for the anti-TIM-3 antibody or the antigen-binding fragment thereof, and the anti-TIM-3 antibody or the antigen-binding fragment thereof is administered during each treatment cycle; one treatment cycle is every 2 weeks for the anti-PD-1 antibody or the antigen-binding fragment thereof, and the anti-PD-1 antibody or the antigen-binding fragment thereof is administered during each treatment cycle. In some embodiments, in the use and the method of the present disclosure, one treatment cycle is every 3 weeks for the anti-TIM-3 antibody or the antigen-binding fragment thereof, and the anti-TIM-3 antibody or the antigen-binding fragment thereof is administered once during each treatment cycle; one treatment cycle is every 2 weeks for the anti-PD-1 antibody or the antigen-binding fragment thereof, and the anti-PD-1 antibody or the antigen-binding fragment thereof is administered once during each treatment cycle. In some embodiments, in the use and the method of the present disclosure, one treatment cycle is every 3 weeks for the anti-TIM-3 antibody or the antigen-binding fragment thereof, and the anti-TIM-3 antibody or the antigen-binding fragment thereof is administered on day 1 of each treatment cycle; one treatment cycle is every 2 weeks for the anti-PD-1 antibody or the antigen-binding fragment thereof, and the anti-PD-1 antibody or the antigen-binding fragment thereof is administered on day 1 of each treatment cycle. In some embodiments, in the use and the method of the present disclosure, one treatment cycle is every 3 weeks for the anti-TIM-3 antibody or the antigen-binding fragment thereof, and the anti-TIM-3 antibody or the antigen-binding fragment thereof is administered once on day 1 of each treatment cycle; one treatment cycle is every 2 weeks for the anti-PD-1 antibody or the antigen-binding fragment thereof, and the anti-PD-1 antibody or the antigen-binding fragment thereof is administered once on day 1 of each treatment cycle. In some specific embodiments, in the use and the method of the present disclosure, one treatment cycle is every 3 weeks for the anti-TIM-3 antibody or the antigen-binding fragment thereof, and about 600 mg or about 1200 mg of the anti-TIM-3 antibody or the antigen-binding fragment thereof is administered during each treatment cycle; one treatment cycle is every 2 weeks for the anti-PD-1 antibody or the antigen-binding fragment thereof, and about 200 mg of the anti-PD-1 antibody or the antigen-binding fragment thereof is administered during each treatment cycle. In some specific embodiments, in the use and the method of the present disclosure, one treatment cycle is every 3 weeks for the anti-TIM-3 antibody or the antigen-binding fragment thereof, and about 600 mg or about 1200 mg of the anti-TIM-3 antibody or the antigen-binding fragment thereof is administered on day 1 of each treatment cycle; one treatment cycle is every 2 weeks for the anti-PD-1 antibody or the antigen-binding fragment thereof, and about 200 mg of the anti-PD-1 antibody or the antigen-binding fragment thereof is administered on day 1 of each treatment cycle.

In some embodiments, the anti-TIM-3 antibody or the antigen-binding fragment thereof and the anti-PD-1 antibody or the antigen-binding fragment thereof are administered to the subject at a mass ratio of the anti-TIM-3 antibody or the antigen-binding fragment thereof to the anti-PD-1 antibody or the antigen-binding fragment thereof of (0.1-180):1, (0.2-50):1, (0.2-9):1, (0.5-9):1, or (3-6):1 in each treatment cycle. In some embodiments, the anti-TIM-3 antibody or the antigen-binding fragment thereof and the anti-PD-1 antibody or the antigen-binding fragment thereof are administered in multiple doses or a single dose. In some embodiments, the anti-TIM-3 antibody or the antigen-binding fragment thereof and the anti-PD-1 antibody or the antigen-binding fragment thereof are both administered in multiple doses.

In some embodiments, in the use and the method of the present disclosure, the anti-TIM-3 antibody or the antigen-binding fragment thereof may be administered to the subject at a dose of 0.01 to 50 mg/kg, 0.1 to 40 mg/kg, 0.1 to 35 mg/kg, 0.1 to 30 mg/kg, 0.1 to 25 mg/kg, 0.1 to 20 mg/kg, 0.1 to 15 mg/kg, 0.1 to 10 mg/kg, 1 to 40 mg/kg, 1 to 35 mg/kg, 1 to 30 mg/kg, 1 to 25 mg/kg, 1 to 20 mg/kg, 1 to 15 mg/kg, 1 to 10 mg/kg, 1 to 3 mg/kg, 3 to 40 mg/kg, 3 to 35 mg/kg, 3 to 30 mg/kg, 3 to 25 mg/kg, 3 to 20 mg/kg, 3 to 15 mg/kg, 3 to 10 mg/kg, 10 to 40 mg/kg, 10 to 30 mg/kg, 10 to 25 mg/kg, or 20 to 25 mg/kg; or the anti-TIM-3 antibody or the antigen-binding fragment thereof may be administered to the subject at a flat dose of 1-2400 mg, 20-1800 mg, 100-1800 mg, 300-1800 mg, 600-1600 mg, 700-1600 mg, 800-1600 mg, 900-1600 mg, 1000-1600 mg, 1100-1600 mg, 1200-1600 mg, 1300-1600 mg, 1400-1600 mg, 1500-1600 mg, 600-1500 mg, 800-1500 mg, 1000-1500 mg, 1200-1500 mg, 600-1200 mg, 800-1200 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, or about 1800 mg.

In some embodiments, in the use and the method of the present disclosure, the anti-PD-1 antibody or the antigen-binding fragment thereof may be administered to the subject at a dose of 0.01 to 50 mg/kg, 0.1 to 40 mg/kg, 0.1 to 35 mg/kg, 0.1 to 30 mg/kg, 0.1 to 25 mg/kg, 0.1 to 20 mg/kg, 0.1 to 15 mg/kg, 0.1 to 10 mg/kg, 1 to 30 mg/kg, 1 to 25 mg/kg, 1 to 20 mg/kg, 1 to 15 mg/kg, 1 to 10 mg/kg, 1 to 8 mg/kg, 1 to 5 mg/kg, 1 to 3 mg/kg, 3 to 30 mg/kg, 3 to 25 mg/kg, 3 to 20 mg/kg, 3 to 15 mg/kg, 3 to 10 mg/kg, 3 to 8 mg/kg, or 3 to 5 mg/kg; or the anti-PD-1 antibody or the antigen-binding fragment thereof may be administered to the subject at a flat dose of 1-1000 mg, 10-1000 mg, 10-800 mg, 20-800 mg, 40-800 mg, 50-700 mg, 50-600 mg, 50-500 mg, 60-500 mg, 80-500 mg, 100-500 mg, 200-500 mg, 100-500 mg, 100-400 mg, 50-350 mg, 100-300 mg, 50-200 mg, 100-200 mg, about 100 mg, or about 200 mg.

In some embodiments, in the use and the method of the present disclosure, the administration regimen (e.g., administration cycle, administration dose, and dose adjustment) for the anti-TIM-3 antibody or the antigen-binding fragment thereof and the anti-PD-1 antibody or the antigen-binding fragment thereof may be adjusted according to the severity of the disease, the response to the disease, any treatment-related toxicity, and the age and health condition of a patient. For example, one treatment cycle for the anti-TIM-3 antibody or the antigen-binding fragment thereof and/or the anti-PD-1 antibody or the antigen-binding fragment thereof may be adjusted to 4 weeks, 5 weeks, 6 weeks, 7 weeks, 8 weeks, 9 weeks, 10 weeks, 11 weeks, or 12 weeks.

The method for the treatment of the present disclosure may be applied to a first-line treatment, a second-line treatment, a third-line treatment, or a later-line treatment. A line of treatment refers to a position in the sequence in which the patient receives different medications or other treatments. The first-line treatment is the first or standard choice of treatment, while the second-line treatment and the third-line treatment are administered after the first-line treatment and after the second-line treatment, respectively. Thus, the first-line treatment is the first treatment for a disease or a condition. In tumor patients, the first-line treatment, sometimes referred to as an initial treatment, may be surgery, chemotherapy, radiation therapy, or a combination thereof. If a patient does not exhibit a positive clinical outcome from the first-line treatment or the second-line treatment, or exhibits a positive clinical response but later experiences disease progression or recurrence, or the current condition is resistant to a previous treatment that previously caused a positive clinical response, the patient will receive a subsequent treatment (e.g., second-line treatment or third-line treatment). Non-limiting examples of the clinical outcome include tumor response, overall survival, progression-free survival, disease-free survival, time to tumor recurrence, time to tumor progression, relative risks, toxicity, or side effects.

In some embodiments, the method for the treatment of the present disclosure is applied to a posterior-line treatment, particularly a second-line treatment or a later-line treatment of a tumor. There is no limitation on the number of previous treatments as long as the entity has been treated for at least one treatment cycle of previous tumor treatment. In some embodiments, the method for the treatment of the present disclosure is applied to a second-line treatment or a later-line treatment of lymphoma. In some embodiments, the method for the treatment of the present disclosure is applied to a third-line treatment or a later-line treatment of lymphoma.

### Anti-TIM-3 antibody or antigen-binding fragment thereof

In some embodiments, the anti-TIM-3 antibody or the antigen-binding fragment thereof of the present disclosure comprises: a heavy chain CDR1 (HCDR1) of the amino acid sequence set forth in SEQ ID NO: 1 or 21, HCDR2 of the amino acid sequence set forth in SEQ ID NO: 2 or 22, HCDR3 of the amino acid sequence set forth in SEQ ID NO: 3 or 23, a light chain CDR1 (LCDR1) of the amino acid sequence set forth in SEQ ID NO: 4 or 24, LCDR2 of the amino acid sequence set forth in SEQ ID NO: 5 or 25, and LCDR3 of the amino acid sequence set forth in SEQ ID NO: 6 or 26. In some embodiments, the anti-TIM-3 antibody or the antigen-binding fragment thereof of the present disclosure comprises: HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6. In some embodiments, the anti-TIM-3 antibody or the antigen-binding fragment thereof of the present disclosure comprises: HCDR1 of the amino acid sequence set forth in SEQ ID NO: 21, HCDR2 of the amino acid sequence set forth in SEQ ID NO: 22, HCDR3 of the amino acid sequence set forth in SEQ ID NO: 23, LCDR1 of the amino acid sequence set forth in SEQ ID NO: 24, LCDR2 of the amino acid sequence set forth in SEQ ID NO: 25, and LCDR3 of the amino acid sequence set forth in SEQ ID NO: 26. The CDR sequences of the anti-TIM-3 antibody or the antigen-binding fragment thereof are shown in Table 1.

**Table 1. CDR sequences of the anti-TIM-3 antibody or the antigen-binding fragment thereof**

| | | |
|---|---|---|
| HCDR1 | GYSFTGYTIN | SEQ ID NO:1 |
| | GFNIKDYYMH | SEQ ID NO:21 |
| HCDR2 | LFNPYNGGTT | SEQ ID NO:2 |
| | WIDPENDNTIY | SEQ ID NO:22 |
| HCDR3 | RYYGYDAMDY | SEQ ID NO:3 |
| | ARDFGYVAWLVY | SEQ ID NO:23 |
| LCDR1 | KSSQSVLYSSNQKNHLA | SEQ ID NO:4 |
| | KASQNVDTAVA | SEQ ID NO:24 |
| LCDR2 | WASTRES | SEQ ID NO:5 |
| | SASNRYT | SEQ ID NO:25 |
| LCDR3 | HQYLSSYT | SEQ ID NO:6 |
| | QQYSSYPT | SEQ ID NO:26 |

It will be appreciated by those skilled in the art that, unless otherwise specified, the term "CDR" or "complementarity determining region" of a given antibody or a region thereof (e.g., a variable region) will be understood to encompass complementarity determining regions defined by any one of the known schemes. Although Table 1 has shown the CDR sequences (wherein the CDRs set forth in SEQ ID NOs: 1-6 are defined by the AbM numbering system), when reference is made to an antibody defined by a specific CDR sequence, the scope of the antibody also encompasses antibodies defined by CDR sequences that are converted to other arbitrary numbering system definitions (e.g., one of or a combination of several of the definitions well known in the art, such as Kabat, Chothia, IMGT, CCG, Contact, etc.). For example, anti-TIM-3 antibodies or antigen-binding fragments thereof comprising the following CDR amino acid sequences are also encompassed within the scope of the anti-TIM-3 antibody or the antigen-binding fragment thereof of the present disclosure: HCDR1 of the amino acid sequence set forth in SEQ ID NO: 1, HCDR2 of the amino acid sequence set forth in SEQ ID NO: 2, HCDR3 of the amino acid sequence set forth in ARRYYGYDAMDY (SEQ ID NO: 31), LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6.

In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7 or 27, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8 or 28. In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 27, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 28. In some embodiments, the anti-TIM-3 antibody or the antigen-binding fragment thereof comprises a heavy chain variable region of the amino acid sequence set forth in SEQ ID NO: 7, and a light chain variable region of the amino acid sequence set forth in SEQ ID NO: 8. In some specific embodiments, the amino acid sequence of the heavy chain variable region of the anti-TIM-3 antibody or the antigen-binding fragment thereof is set forth in SEQ ID NO: 7, and the amino acid sequence of the light chain variable region of the anti-TIM-3 antibody or the antigen-binding fragment thereof is set forth in SEQ ID NO: 8. In some embodiments, the anti-TIM-3 antibody or the antigen-binding fragment thereof comprises a heavy chain variable region of the amino acid sequence set forth in SEQ ID NO: 27, and a light chain variable region of the amino acid sequence set forth in SEQ ID NO: 28. In some specific embodiments, the amino acid sequence of the heavy chain variable region of the anti-TIM-3 antibody or the antigen-binding fragment thereof is set forth in SEQ ID NO: 27, and the amino acid sequence of the light chain variable region of the anti-TIM-3 antibody or the antigen-binding fragment thereof is set forth in SEQ ID NO: 28.

QVQLVQSGAEVKKPGASVKVSCKASGYSFTGYTINWVRQAPGQGLEWMGLFNPYNGGTTYAQKFQGR
VTMTVDKSTSTVYMELSSLRSEDTAVYYCARRYYGYDAMDYWGQGTLVTVSS (SEQ ID NO: 7);

DIQMTQSPSSLSASVGDRVTITCKSSQSVLYSSNQKNHLAWYQQKPGKAPKLLIYWASTRESGVPSRFSGS
GSGTDFTLTISSLQPEDFATYYCHQYLSSYTFGQGTKLEIK (SEQ ID NO: 8);

EVQLQQSGAEVVRPGALVKLSCKASGFNIKDYYMHWVRQRPEQGLEWIGWIDPENDNTIYDPKFQDRA
SITADTSSNTAYLQLSSLTSEDTAVYYCARDFGYVAWLVYWGQGTLVTVSA (SEQ ID NO: 27);

DIVMTQSQEFMSTSVGDRVSITCKASQNVDTAVAWYQQKPGQSPKLLIYSASNRYTGVPDRFTGTGSGTD
FTLTINNMQSEDLADYFCQQYSSYPTFGGRTKLEIK (SEQ ID NO:28).

In some embodiments, the anti-TIM-3 antibody or the antigen-binding fragment thereof comprises: HCDR1 comprising the amino acid sequence set forth in SEQ ID NO: 1, HCDR2 comprising the amino acid sequence set forth in SEQ ID NO: 2, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6; and the anti-TIM-3 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8.

In some embodiments, the anti-TIM-3 antibody or the antigen-binding fragment thereof may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, and IgG4, or other types of immunoglobulins, preferably a heavy chain of IgG4. In some embodiments, the light chain constant region is derived from a human immunoglobulin light chain, for example, a κ light chain or a λ light chain of a human immunoglobulin. In some embodiments, the constant region may comprise any modification described in the text, for example, insertion, deletion, substitution, or chemical modification of amino acids. In some embodiments, the constant region comprises a mutation that alters the effector function. In some embodiments, any amino acid residue of the constant region may be substituted by an amino acid residue of any allotype.

In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 9 or 29, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 10 or 30. In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 9, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 10. In some embodiments, the anti-TIM-3 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 29, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 30. In some embodiments, the anti-TIM-3 antibody or the antigen-binding fragment thereof comprises a heavy chain of the amino acid sequence set forth in SEQ ID NO: 9, and a light chain of the amino acid sequence set forth in SEQ ID NO: 10. In some specific embodiments, the amino acid sequence of the heavy chain of the anti-TIM-3 antibody or the antigen-binding fragment thereof is set forth in SEQ ID NO: 9, and the amino acid sequence of the light chain of the anti-TIM-3 antibody or the antigen-binding fragment thereof is set forth in SEQ ID NO: 10. In some embodiments, the anti-TIM-3 antibody or the antigen-binding fragment thereof comprises a heavy chain of the amino acid sequence set forth in SEQ ID NO: 29, and a light chain of the amino acid sequence set forth in SEQ ID NO: 30. In some specific embodiments, the amino acid sequence of the heavy chain of the anti-TIM-3 antibody or the antigen-binding fragment thereof is set forth in SEQ ID NO: 29, and the amino acid sequence of the light chain of the anti-TIM-3 antibody or the antigen-binding fragment thereof is set forth in SEQ ID NO: 30.

QVQLVQSGAEVKKPGASVKVSCKASGYSFTGYTINWVRQAPGQGLEWMGLFNPYNGGTTYAQKFQGR
VTMTVDKSTSTVYMELSSLRSEDTAVYYCARRYYGYDAMDYWGQGTLVTVSSASTKGPSVFPLAPCSRS
TSESTAALGCLVKDYFPEPVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCVVVDVSQEDPEVQFNWYV
DGVEVHNAKTKPREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYSRLTVDKSRWQ
EGNVFSCSVMHEALHNHYTQKSLSLSLGK (SEQ ID NO: 9);

DIQMTQSPSSLSASVGDRVTITCKSSQSVLYSSNQKNHLAWYQQKPGKAPKLLIYWASTRESGVPSRFSGS
GSGTDFTLTISSLQPEDFATYYCHQYLSSYTFGQGTKLEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC (SEQ ID NO:10);

EVQLQQSGAEVVRPGALVKLSCKASGFNIKDYYMHWVRQRPEQGLEWIGWIDPENDNTIYDPKFQDRA
SITADTSSNTAYLQLSSLTSEDTAVYYCARDFGYVAWLVYWGQGTLVTVSAASTKGPSVFPLAPCSRSTSE
STAALGCLVKDYFPEPVTVSWNSGALTSGVHTFPAVLQSSGLYSLSSVVTVPSSSLGTKTYTCNVDHKPSN
TKVDKRVESKYGPPCPPCPAPEFLGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSRLTVDKSRWQEG
NVFSCSVMHEALHNHYTQKSLSLSLGK (SEQ ID NO:29);

DIVMTQSQEFMSTSVGDRVSITCKASQNVDTAVAWYQQKPGQSPKLLIYSASNRYTGVPDRFTGTGSGTD
FTLTINNMQSEDLADYFCQQYSSYPTFGGRTKLEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNRG
EC (SEQ ID NO: 30).

In some embodiments, the anti-TIM-3 antibody or the antigen-binding fragment thereof of the present disclosure is mAb 50B5 or an antigen-binding fragment thereof described in a patent application with publication No. WO2020041520 or CN112566936, or a chimeric antibody of mAb 50B5 or an antigen-binding fragment thereof, or a humanized antibody of mAb 50B5 or an antigen-binding fragment thereof.

In other embodiments, the anti-TIM-3 antibody or the antigen-binding fragment thereof of the present disclosure is mAb 15B4 or an antigen-binding fragment thereof described in a patent application with publication No. WO2020041520 or CN112566936, or a chimeric antibody of mAb 15B4 or an antigen-binding fragment thereof, or a humanized antibody of mAb 15B4 or an antigen-binding fragment thereof.

In other embodiments, the anti-TIM-3 antibody or the antigen-binding fragment thereof of the present disclosure is selected from the group consisting of sabatolimab, cobolimab, surzebiclimab, RG-7769 of Roche, SHR-1702 of Hengrui Pharmaceuticals, INCAGN-2390 of Agenus, BC-3402 of WuXi Biologics, LBL-003 of Leads Biolabs, LNL-005 of L&L Biopharma, or BMS-986258 of BMS.

### Anti-PD-1 antibody or antigen-binding fragment thereof

In some embodiments, the anti-PD-1 antibody or the antigen-binding fragment thereof of the present disclosure comprises: HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16. The CDR sequences of the anti-PD-1 antibody or the antigen-binding fragment thereof are shown in Table 2.

**Table 2. CDR sequences of the anti-PD-1 antibody or the antigen-binding fragment thereof**

| | | |
|---|---|---|
| HCDR1 | GFAFSSYD | SEQ ID NO:11 |
| HCDR2 | ISGGGRYT | SEQ ID NO:12 |
| HCDR3 | ANRYGEAWFAY | SEQ ID NO:13 |
| LCDR1 | QDINTY | SEQ ID NO:14 |
| LCDR2 | RAN | SEQ ID NO:15 |
| LCDR3 | LQYDEFPLT | SEQ ID NO:16 |

It will be appreciated by those skilled in the art that, unless otherwise specified, the term "CDR" or "complementarity determining region" of a given antibody or a region thereof (e.g., a variable region) will be understood to encompass complementarity determining regions defined by any one of the known schemes. Although Table 2 has shown the CDRs, when reference is made to an antibody defined by a specific CDR sequence, the scope of the antibody also encompasses antibodies defined by CDR sequences that are converted to other arbitrary numbering system definitions (e.g., one of or a combination of several of the definitions well known in the art, such as AbM, Kabat, Chothia, CCG, Contact, etc.).

In some embodiments, the anti-PD-1 antibody or the antigen-binding fragment thereof comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18. In some embodiments, the anti-PD-1 antibody or the antigen-binding fragment thereof comprises a heavy chain variable region of the amino acid sequence set forth in SEQ ID NO: 17, and a light chain variable region of the amino acid sequence set forth in SEQ ID NO: 18. In some specific embodiments, the amino acid sequence of the heavy chain variable region of the anti-PD-1 antibody or the antigen-binding fragment thereof is set forth in SEQ ID NO: 17, and the amino acid sequence of the light chain variable region of the anti-PD-1 antibody or the antigen-binding fragment thereof is set forth in SEQ ID NO: 18.

EVQLVESGGGLVQPGGSLRLSCAASGFAFSSYDMSWVRQAPGKGLDWVATISGGGRYTYYPDSVKGRFT
ISRDNSKNNLYLQMNSLRAEDTALYYCANRYGEAWFAYWGQGTLVTVSS (SEQ ID NO: 17);

DIQMTQSPSSMSASVGDRVTFTCRASQDINTYLSWFQQKPGKSPKTLIYRANRLVSGVPSRFSGSGSGQD
YTLTISSLQPEDMATYYCLQYDEFPLTFGAGTKLELK (SEQ ID NO: 18).

In some embodiments, the anti-PD-1 antibody or the antigen-binding fragment thereof may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, and IgG4, or other types of immunoglobulins, preferably a heavy chain of IgG1. In some embodiments, the light chain constant region is derived from a human immunoglobulin light chain, for example, a κ light chain or a λ light chain of a human immunoglobulin. In some embodiments, the constant region may comprise any modification described in the text, for example, insertion, deletion, substitution, or chemical modification of amino acids. In some embodiments, the constant region comprises a mutation that alters the effector function. In some embodiments, any amino acid residue of the constant region may be substituted by an amino acid residue of any allotype.

In some embodiments, the anti-PD-1 antibody or the antigen-binding fragment thereof comprises a heavy chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19, and a light chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20. In some embodiments, the anti-PD-1 antibody or the antigen-binding fragment thereof comprises a heavy chain of the amino acid sequence set forth in SEQ ID NO: 19, and a light chain of the amino acid sequence set forth in SEQ ID NO: 20. In some specific embodiments, the amino acid sequence of the heavy chain of the anti-PD-1 antibody or the antigen-binding fragment thereof is set forth in SEQ ID NO: 19, and the amino acid sequence of the light chain of the anti-PD-1 antibody or the antigen-binding fragment thereof is set forth in SEQ ID NO: 20.

EVQLVESGGGLVQPGGSLRLSCAASGFAFSSYDMSWVRQAPGKGLDWVATISGGGRYTYYPDSVKGRFT
ISRDNSKNNLYLQMNSLRAEDTALYYCANRYGEAWFAYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNT
KVDKKVEPKSCDKTHTCPPCPAPEAAGAPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQV
YTLPPSRDELTKNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 19);

DIQMTQSPSSMSASVGDRVTFTCRASQDINTYLSWFQQKPGKSPKTLIYRANRLVSGVPSRFSGSGSGQD
YTLTISSLQPEDMATYYCLQYDEFPLTFGAGTKLELKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC (SEQ ID NO: 20).

In other embodiments, the anti-PD-1 antibody or the antigen-binding fragment thereof of the present disclosure is selected from the group consisting of nivolumab, pembrolizumab, toripalimab, sintilimab, camrelizumab, tislelizumab, zimberelimab, balstilimab, geptanolimab, LZM009 of Livzon, cemiplimab, serplulimab, prolgolimab, pucotenlimab (HX008), nofazinlimab, finotonlimab, dostarlimab, cetrelimab, QL1604 of Qilu Pharmaceutical, spartalizumab, retifanlimab, sasanlimab, F520 of Shandong New Time Pharmaceutical, or SG001 of Sumgen Bio.

### Pharmaceutical composition comprising antibody or antigen-binding fragment thereof

In some embodiments, the anti-TIM-3 antibody or the antigen-binding fragment thereof and/or the anti-PD-1 antibody or the antigen-binding fragment thereof are formulated into a formulation for administration by a parenteral route. In some specific embodiments, the anti-TIM-3 antibody or the antigen-binding fragment thereof and/or the anti-PD-1 antibody or the antigen-binding fragment thereof are formulated into a formulation for administration by intravenous, intramuscular, subcutaneous, or other parenteral routes, for example, injection or infusion. In some specific embodiments, the anti-TIM-3 antibody or the antigen-binding fragment thereof and/or the anti-PD-1 antibody or the antigen-binding fragment thereof are formulated into a formulation for intravenous, intramuscular, or subcutaneous administration. In some specific embodiments, the anti-TIM-3 antibody or the antigen-binding fragment thereof and/or the anti-PD-1 antibody or the antigen-binding fragment thereof are formulated into a formulation for intravenous injection or infusion.

The anti-TIM-3 antibody or the antigen-binding fragment thereof and/or the anti-PD-1 antibody or the antigen-binding fragment thereof may be formulated into a suitable dosage form, including, but not limited to, a tablet, a lozenge, a pill, a capsule (e.g., hard capsule, soft capsule, enteric capsule, or microcapsule), an elixir, a granule, a syrup, an injection (i.e., formulation suitable for injection, e.g., by intramuscular, intravenous, intraperitoneal or subcutaneous route), a granule, an emulsion, a suspension, a solution, a dispersant, and a dosage form of a sustained-released preparation for oral or non-oral administration. In some specific embodiments, the anti-TIM-3 antibody or the antigen-binding fragment thereof and/or the anti-PD-1 antibody or the antigen-binding fragment thereof may be formulated into an injection. In some specific embodiments, the anti-TIM-3 antibody or the antigen-binding fragment thereof and/or the anti-PD-1 antibody or the antigen-binding fragment thereof may be formulated into a formulation suitable for intravenous injection.

In some embodiments, the anti-TIM-3 antibody or the antigen-binding fragment thereof and the anti-PD-1 antibody or the antigen-binding fragment thereof are formulated in a single formulation. In some embodiments, the anti-TIM-3 antibody or the antigen-binding fragment thereof and the anti-PD-1 antibody or the antigen-binding fragment thereof are formulated together with one or more pharmaceutically acceptable excipients to prepare a suitable pharmaceutical composition.

In some embodiments, the anti-TIM-3 antibody or the antigen-binding fragment thereof and the anti-PD-1 antibody or the antigen-binding fragment thereof are formulated separately (i.e., each present in the form of a pharmaceutical composition). In some embodiments, the anti-TIM-3 antibody or the antigen-binding fragment thereof is formulated together with one or more pharmaceutically acceptable excipients to prepare a suitable pharmaceutical composition. In some embodiments, the anti-PD-1 antibody or the antigen-binding fragment thereof is formulated together with one or more pharmaceutically acceptable excipients to prepare a suitable pharmaceutical composition.

In some embodiments, the unit dose of the pharmaceutical composition comprising the anti-TIM-3 antibody or the antigen-binding fragment thereof is 60-1800 mg, 100-1600 mg, 120-1600 mg, 180-1200 mg, or 240-600 mg of the anti-TIM-3 antibody or the antigen-binding fragment thereof, for example, about 60 mg, about 120 mg, about 160 mg, about 180 mg, about 200 mg, about 240 mg, about 280 mg, about 300 mg, about 320 mg, about 360 mg, about 400 mg, about 420 mg, about 440 mg, about 480 mg, about 520 mg, about 540 mg, about 560 mg, about 600 mg, about 640 mg, about 660 mg, about 680 mg, about 720 mg, about 760 mg, about 780 mg, about 800 mg, about 840 mg, about 880 mg, about 900 mg, about 920 mg, about 960 mg, about 1000 mg, about 1020 mg, about 1040 mg, about 1080 mg, about 1120 mg, about 1140 mg, about 1160 mg, about 1200 mg, about 1240 mg, about 1260 mg, about 1280 mg, about 1320 mg, about 1360 mg, about 1380 mg, about 1400 mg, about 1440 mg, about 1480 mg, about 1500 mg, about 1520 mg, about 1560 mg, about 1600 mg, about 1620 mg, about 1640 mg, about 1680 mg, about 1720 mg, about 1740 mg, about 1760 mg, and/or about 1800 mg, or a range formed by any of the above values of the anti-TIM-3 antibody or the antigen-binding fragment thereof.

In some embodiments, the unit dose of the pharmaceutical composition comprising the anti-PD-1 antibody or the antigen-binding fragment thereof is 10-500 mg, 50-500 mg, 50-200 mg, or 100-200 mg of the anti-PD-1 antibody or the antigen-binding fragment thereof, for example, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, and/or about 500 mg, or a range formed by any of the above values of the anti-PD-1 antibody or the antigen-binding fragment thereof.

As used herein, "excipient" is used to describe any ingredient other than the active ingredient. The selection of an excipient will largely depend on factors such as the particular mode of administration, the efficacy of the excipient on solubility and stability, and the nature of the dosage form. As used herein, "pharmaceutically acceptable excipient" includes, but is not limited to, an excipient, a diluent, a filler, a binder, a disintegrant, a solubilizer, a stabilizer, a coloring agent, a flavoring agent, a surfactant, an emulsifier, a buffering agent, or an encapsulating material. The amount of each excipient used may vary within the conventional range in the art.

In a specific embodiment, the pharmaceutical composition comprising the anti-TIM-3 antibody or the antigen-binding fragment thereof is a water-soluble injection. In a specific embodiment, the pharmaceutical composition comprising the anti-PD-1 antibody or the antigen-binding fragment thereof is a water-soluble injection. In a specific embodiment, the single formulation comprising the anti-TIM-3 antibody or the antigen-binding fragment thereof and the anti-PD-1 antibody or the antigen-binding fragment thereof is a water-soluble injection. The water-soluble injection includes, but is not limited to, a non-lyophilized water-soluble formulation or a water-soluble formulation reconstituted from a lyophilized powder.

In other embodiments, the pharmaceutical composition comprising the anti-TIM-3 antibody or the antigen-binding fragment thereof is a lyophilized formulation. In other embodiments, the pharmaceutical composition comprising the anti-PD-1 antibody or the antigen-binding fragment thereof is a lyophilized formulation. In other embodiments, the single formulation comprising the anti-TIM-3 antibody or the antigen-binding fragment thereof and the anti-PD-1 antibody or the antigen-binding fragment thereof is a lyophilized formulation. The lyophilized formulation refers to a formulation prepared by subjecting an aqueous solution to a lyophilization process in which the substance is first frozen, and then the amount of the solvent is reduced by sublimation (primary drying process) and then by desorption (secondary drying process) until the amount of the solvent is reduced to a value that no longer supports the biological activity or a chemical reaction. The lyophilized formulation of the present disclosure may also be dried by other methods known in the art, such as spray drying and bubble drying.

In some embodiments, the anti-PD-1 antibody or the antigen-binding fragment thereof in the pharmaceutical composition comprising the anti-PD-1 antibody or the antigen-binding fragment thereof has a concentration of 0.1-50 mg/mL, 0.5-30 mg/mL, 0.8-20 mg/mL, 1-15 mg/mL, 1-10 mg/mL, 1-5 mg/mL, 2-20 mg/mL, 2-15 mg/mL, 2-10 mg/mL, or 2-5 mg/mL. In some specific embodiments, the anti-PD-1 antibody or the antigen-binding fragment thereof has a concentration of about 0.8 mg/mL, about 1 mg/mL, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 15 mg/mL, or about 20 mg/mL. In some embodiments, the anti-PD-1 antibody or the antigen-binding fragment thereof has a concentration of about 1 mg/mL. In some embodiments, the anti-PD-1 antibody or the antigen-binding fragment thereof has a concentration of about 2 mg/mL. In some embodiments, the anti-PD-1 antibody or the antigen-binding fragment thereof has a concentration of about 5 mg/mL. In some embodiments, the anti-PD-1 antibody or the antigen-binding fragment thereof has a concentration of about 10 mg/mL.

In some embodiments, the anti-PD-1 antibody or the antigen-binding fragment thereof is present in the form of a pharmaceutical composition comprising the anti-PD-1 antibody or the antigen-binding fragment thereof, a buffering agent, an isotonicity modifier/stabilizer, and a surfactant. In a specific embodiment, the anti-PD-1 antibody or the antigen-binding fragment thereof is present in the form of a liquid pharmaceutical composition (e.g., an injection) comprising the anti-PD-1 antibody or the antigen-binding fragment thereof, sodium acetate trihydrate, glacial acetic acid, sorbitol, and polysorbate 80.

### Mode of administration

The content below is not intended to limit the mode of administration of the pharmaceutical combination of the present disclosure.

The components in the pharmaceutical combination of the present disclosure may be independently administered, or some or all of the components are co-administered, by various suitable routes, including, but not limited to, oral administration or parenteral administration (by intravenous, intramuscular, topical or subcutaneous route). In some embodiments, the components in the pharmaceutical combination of the present disclosure may be independently administered, or some or all of the components are co-administered, orally or by injection, for example, intravenous injection or subcutaneous injection.

The components in the pharmaceutical combination of the present disclosure may be independently formulated into suitable dosage forms, or some or all of the components are co-formulated into a suitable dosage form, including, but not limited to, a tablet, a lozenge, a pill, a capsule (e.g., hard capsule, soft capsule, enteric capsule, or microcapsule), an elixir, a granule, a syrup, an injection (i.e., formulation suitable for injection, e.g., an injection suitable for intramuscular, intravenous, intraperitoneal or subcutaneous administration), a granule, an emulsion, a suspension, a solution, a dispersant, and a dosage form of a sustained-released preparation for oral or non-oral administration. In some embodiments, the components in the pharmaceutical combination of the present disclosure may be independently formulated into injections, or some or all of the components are co-formulated into an injection.

The components in the pharmaceutical combination of the present disclosure may be independently formulated with a pharmaceutically acceptable excipient (including a carrier and/or an excipient), or some or all of the components are co-formulated with a pharmaceutically acceptable excipient (including a carrier and/or an excipient).

The pharmaceutical combination of the present disclosure may further comprise an additional therapeutic agent. In one embodiment, the additional therapeutic agent may be a therapeutic agent for a tumor known in the art.

### Tumor

In certain aspects, the tumor of the present disclosure is a malignant tumor (i.e., cancer); the malignant tumor refers to any malignant and/or aggressive growth caused by abnormal cell growth. In some embodiments, the tumor is a non-solid tumor. In some embodiments, the tumor is a treatment-naive, refractory, recurrent, and/or advanced non-solid tumor. In some embodiments, the tumor is a recurrent or refractory non-solid tumor.

In some embodiments, the tumor is lymphoma. In some embodiments, the tumor is treatment-naive, refractory, recurrent, and/or advanced lymphoma. In some embodiments, the tumor is recurrent or refractory lymphoma.

In some embodiments, the tumor is lymphoma that has failed treatment with an immune checkpoint inhibitor. In some embodiments, the tumor is lymphoma resistant to an immune checkpoint inhibitor. In some embodiments, the tumor is recurrent or refractory lymphoma that has failed treatment with an immune checkpoint inhibitor. In some embodiments, the tumor is recurrent or refractory lymphoma resistant to an immune checkpoint inhibitor. In some embodiments, the tumor is recurrent or refractory lymphoma that has failed treatment with a PD-1 or PD-L1 inhibitor. In some embodiments, the tumor is recurrent or refractory lymphoma resistant to a PD-1 or PD-L1 inhibitor.

In some embodiments, the lymphoma is Hodgkin's lymphoma. In some embodiments, the lymphoma is recurrent or refractory Hodgkin's lymphoma. In some embodiments, the lymphoma is Hodgkin's lymphoma that has failed treatment with an immune checkpoint inhibitor. In some embodiments, the lymphoma is Hodgkin's lymphoma resistant to an immune checkpoint inhibitor. In some embodiments, the lymphoma is recurrent or refractory Hodgkin's lymphoma that has failed treatment with an immune checkpoint inhibitor. In some embodiments, the lymphoma is recurrent or refractory Hodgkin's lymphoma resistant to an immune checkpoint inhibitor. In some embodiments, the lymphoma is Hodgkin's lymphoma that has failed treatment with a PD-1 or PD-L1 inhibitor. In some embodiments, the lymphoma is Hodgkin's lymphoma resistant to a PD-1 or PD-L1 inhibitor. In some embodiments, the lymphoma is recurrent or refractory Hodgkin's lymphoma that has failed treatment with a PD-1 or PD-L1 inhibitor. In some embodiments, the lymphoma is recurrent or refractory Hodgkin's lymphoma resistant to a PD-1 or PD-L1 inhibitor.

In some embodiments, the lymphoma is classical Hodgkin's lymphoma. In some embodiments, the lymphoma is recurrent or refractory classical Hodgkin's lymphoma. In some embodiments, the lymphoma is classical Hodgkin's lymphoma that has failed treatment with an immune checkpoint inhibitor. In some embodiments, the lymphoma is classical Hodgkin's lymphoma resistant to an immune checkpoint inhibitor. In some embodiments, the lymphoma is recurrent or refractory classical Hodgkin's lymphoma that has failed treatment with an immune checkpoint inhibitor. In some embodiments, the lymphoma is recurrent or refractory classical Hodgkin's lymphoma resistant to an immune checkpoint inhibitor. In some embodiments, the lymphoma is classical Hodgkin's lymphoma that has failed treatment with a PD-1 or PD-L1 inhibitor. In some embodiments, the lymphoma is classical Hodgkin's lymphoma resistant to a PD-1 or PD-L1 inhibitor. In some embodiments, the lymphoma is recurrent or refractory classical Hodgkin's lymphoma that has failed treatment with a PD-1 or PD-L1 inhibitor. In some embodiments, the lymphoma is recurrent or refractory classical Hodgkin's lymphoma resistant to a PD-1 or PD-L1 inhibitor.

In some embodiments, the lymphoma is nodular lymphocyte-predominant Hodgkin's lymphoma.

In some embodiments, the lymphoma is non-Hodgkin's lymphoma. In some embodiments, the lymphoma is T-cell non-Hodgkin's lymphoma or B-cell non-Hodgkin's lymphoma. In some embodiments, the lymphoma is aggressive non-Hodgkin's lymphoma or indolent non-Hodgkin's lymphoma. In some embodiments, the lymphoma is recurrent or refractory non-Hodgkin's lymphoma. In some embodiments, the lymphoma is recurrent or refractory T-cell non-Hodgkin's lymphoma. In some embodiments, the lymphoma is recurrent or refractory B-cell non-Hodgkin's lymphoma.

In some embodiments, the non-Hodgkin's lymphoma is diffuse large B-cell lymphoma, follicular lymphoma, peripheral T-cell lymphoma, mantle cell lymphoma, Burkitt lymphoma, lymphoblastic lymphoma, cutaneous T-cell lymphoma, cutaneous B-cell lymphoma, marginal zone lymphoma, and/or AIDS-related B-cell lymphoma. In some embodiments, the diffuse large B-cell lymphoma, follicular lymphoma, peripheral T-cell lymphoma, mantle cell lymphoma, Burkitt lymphoma, lymphoblastic lymphoma, cutaneous T-cell lymphoma, cutaneous B-cell lymphoma, marginal zone lymphoma, and/or AIDS-related B-cell lymphoma are recurrent or refractory. In some embodiments, the diffuse large B-cell lymphoma is recurrent or refractory. In some embodiments, the follicular lymphoma is recurrent or refractory. In some embodiments, the peripheral T-cell lymphoma is recurrent or refractory.

In some embodiments, the follicular lymphoma is high-grade follicular lymphoma or low-grade follicular lymphoma. In some embodiments, the follicular lymphoma is recurrent or refractory high-grade follicular lymphoma. In some embodiments, the follicular lymphoma is recurrent or refractory low-grade follicular lymphoma.

In some embodiments, the peripheral T-cell lymphoma is peripheral T-cell lymphoma not otherwise specified (PTCL-NOS), angioimmunoblastic T-cell lymphoma (AITL), extranodal NK/T-cell lymphoma (ENKTL), anaplastic lymphoma kinase (ALK) positive anaplastic large cell lymphoma (ALK⁺ ALCL), or ALK negative anaplastic large cell lymphoma (ALK⁻ ALCL). In some embodiments, the extranodal NK/T-cell lymphoma is extranodal NK/T-cell lymphoma, nasal type (ENKTL-NT), or extranodal NK/T-cell lymphoma, non-nasal type. In some embodiments, the peripheral T-cell lymphoma not otherwise specified (PTCL-NOS), angioimmunoblastic T-cell lymphoma (AITL), extranodal NK/T-cell lymphoma (ENKTL), ALK positive anaplastic large cell lymphoma (ALK⁺ ALCL), and/or ALK negative anaplastic large cell lymphoma (ALK⁻ ALCL) are recurrent or refractory.

In some embodiments, the entity of the lymphoma has not previously been treated for the lymphoma (e.g., lacks an effective treatment). In some embodiments, the entity of the lymphoma has not previously received systemic treatment to treat the lymphoma. In some embodiments, the entity of the lymphoma has not previously received surgery, radiation therapy, chemotherapy, and/or immunotherapy to treat the lymphoma. In some embodiments, the entity of the lymphoma has not previously received immunotherapy to treat the lymphoma. In some specific embodiments, the lymphoma is recurrent or refractory diffuse large B-cell lymphoma, and the entity of the lymphoma has not previously received an immune checkpoint inhibitor (e.g., a PD-1 or PD-L1 inhibitor) to treat the lymphoma. In some specific embodiments, the lymphoma is recurrent or refractory follicular lymphoma, and the entity of the lymphoma has not previously received an immune checkpoint inhibitor (e.g., a PD-1 or PD-L1 inhibitor) to treat the lymphoma. In some specific embodiments, the lymphoma is recurrent or refractory peripheral T-cell lymphoma, and the entity of the lymphoma has not previously received an immune checkpoint inhibitor (e.g., a PD-1 or PD-L1 inhibitor) to treat the lymphoma. In some specific embodiments, the lymphoma is recurrent or refractory non-Hodgkin's lymphoma, and the entity of the lymphoma has not previously received an immune checkpoint inhibitor (e.g., a PD-1 or PD-L1 inhibitor) to treat the lymphoma.

In some embodiments, the entity of the lymphoma has previously been treated for the lymphoma with one or more different anti-tumor therapies (e.g., treatment failure or inapplicability). In some embodiments, the entity of the lymphoma has previously been treated for the lymphoma with one or more of surgery, radiation therapy, chemotherapy, and immunotherapy (e.g., treatment failure or inapplicability).

In some embodiments, the entity of the lymphoma has previously received treatment for the lymphoma with a systemic treatment (e.g., treatment failure). In some embodiments, the entity of the lymphoma has previously received treatment for the lymphoma with at least a first-line systemic treatment (e.g., treatment failure). In some embodiments, the entity of the lymphoma has previously received treatment for the lymphoma with at least a second-line systemic treatment (e.g., treatment failure).

In some embodiments, the lymphoma is recurrent or refractory non-Hodgkin's lymphoma, and the entity of the lymphoma has previously received treatment for the lymphoma with at least a first-line systemic treatment (e.g., treatment failure). In some embodiments, the lymphoma is recurrent or refractory T-cell non-Hodgkin's lymphoma, and the entity of the lymphoma has previously received treatment for the lymphoma with at least a first-line systemic treatment (e.g., treatment failure). In some embodiments, the lymphoma is recurrent or refractory peripheral T-cell lymphoma, cutaneous T-cell lymphoma, or lymphoblastic lymphoma, and the entity of the lymphoma has previously received treatment for the lymphoma with at least a first-line systemic treatment (e.g., treatment failure). In some embodiments, the lymphoma is recurrent or refractory peripheral T-cell lymphoma, and the entity of the lymphoma has previously received treatment for the lymphoma with at least a first-line systemic treatment (e.g., treatment failure).

In some embodiments, the entity of the lymphoma has previously received at least a second-line systemic treatment and is resistant to an immune checkpoint inhibitor (e.g., a PD-1 or PD-L1 inhibitor). In some embodiments, the lymphoma is recurrent or refractory classical Hodgkin's lymphoma resistant to an immune checkpoint inhibitor, and the entity of the lymphoma has previously received treatment for the lymphoma with at least a second-line systemic treatment (e.g., treatment failure). In some embodiments, the lymphoma is recurrent or refractory classical Hodgkin's lymphoma, and the entity of the lymphoma has previously received treatment for the lymphoma with at least a second-line systemic treatment and is resistant to a PD-1 or PD-L1 inhibitor. In some embodiments, the lymphoma is recurrent or refractory classical Hodgkin's lymphoma resistant to a PD-1 or PD-L1 inhibitor, and the entity of the lymphoma has previously received treatment for the lymphoma with at least a second-line systemic treatment (e.g., treatment failure).

In some embodiments, the immune checkpoint (e.g., PD-1 or PD-L1) inhibitor is an antibody directed against an immune checkpoint (e.g., PD-1 or PD-L1) or an antigen-binding fragment thereof, for example, nivolumab, pembrolizumab, toripalimab, sintilimab, camrelizumab, tislelizumab, balstilimab, zimberelimab, atezolizumab, durvalumab, avelumab, envafolimab, or sugemalimab.

In some embodiments, the entity of the lymphoma has previously received at least a second-line systemic treatment and at least one CD20-targeted treatment. In some embodiments, the lymphoma is recurrent or refractory non-Hodgkin's lymphoma, and the entity of the lymphoma has previously received treatment for the lymphoma with at least a second-line systemic treatment and at least one CD20-targeted treatment (e.g., treatment failure). In some embodiments, the lymphoma is recurrent or refractory B-cell non-Hodgkin's lymphoma, and the entity of the lymphoma has previously received treatment for the lymphoma with at least a second-line systemic treatment and at least one CD20-targeted treatment (e.g., treatment failure). In some embodiments, the lymphoma is recurrent or refractory diffuse large B-cell lymphoma, and the entity of the lymphoma has previously received treatment for the lymphoma with at least a second-line systemic treatment and at least one CD20-targeted treatment (e.g., treatment failure). In some embodiments, the lymphoma is recurrent or refractory follicular lymphoma, and the entity of the lymphoma has previously received treatment for the lymphoma with at least a second-line systemic treatment and at least one CD20-targeted treatment (e.g., treatment failure). In some embodiments, the lymphoma is recurrent or refractory mantle cell lymphoma, Burkitt lymphoma, cutaneous B-cell lymphoma, marginal zone lymphoma, lymphoblastic lymphoma or AIDS-related B-cell lymphoma, and the entity of the lymphoma has previously received treatment for the lymphoma with at least a second-line systemic treatment and at least one CD20-targeted treatment (e.g., treatment failure).

In some embodiments, the CD20-targeted treatment includes, but is not limited to, administration of an anti-CD20 antibody or an antigen-binding fragment thereof, a CD20-targeted CAR-T cell, or an anti-CD20 antibody-drug conjugate (ADC), for example, rituximab, obinutuzumab, MRG001, or TRS005.

The present disclosure further provides the following specific embodiments, which, however, are not intended to limit the scope of the present disclosure:
Embodiment 1: A pharmaceutical combination, comprising an anti-TIM-3 antibody or an antigen-binding fragment thereof and an anti-PD-1 antibody or an antigen-binding fragment thereof, wherein the anti-TIM-3 antibody or the antigen-binding fragment thereof comprises: HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6; or
   HCDR1 of the amino acid sequence set forth in SEQ ID NO: 21, HCDR2 of the amino acid sequence set forth in SEQ ID NO: 22, HCDR3 of the amino acid sequence set forth in SEQ ID NO: 23, LCDR1 of the amino acid sequence set forth in SEQ ID NO: 24, LCDR2 of the amino acid sequence set forth in SEQ ID NO: 25, and LCDR3 of the amino acid sequence set forth in SEQ ID NO: 26.
Embodiment 2: The pharmaceutical combination according to embodiment 1, wherein the anti-TIM-3 antibody or the antigen-binding fragment thereof comprises: a heavy chain variable region having an amino acid sequence having at least 95% identity to the amino acid sequence set forth in SEQ ID NO: 7, and a light chain variable region having an amino acid sequence having at least 95% identity to the amino acid sequence set forth in SEQ ID NO: 8; or
   a heavy chain variable region having an amino acid sequence having at least 95% identity to the amino acid sequence set forth in SEQ ID NO: 27, and a light chain variable region having an amino acid sequence having at least 95% identity to the amino acid sequence set forth in SEQ ID NO: 28.
Embodiment 3: The pharmaceutical combination according to embodiment 1 or 2, wherein the anti-TIM-3 antibody or the antigen-binding fragment thereof comprises: a heavy chain having an amino acid sequence having at least 95% identity to the amino acid sequence set forth in SEQ ID NO: 9, and a light chain having an amino acid sequence having at least 95% identity to the amino acid sequence set forth in SEQ ID NO: 10; or a heavy chain having an amino acid sequence having at least 95% identity to the amino acid sequence set forth in SEQ ID NO: 29, and a light chain having an amino acid sequence having at least 95% identity to the amino acid sequence set forth in SEQ ID NO: 30.
Embodiment 4: The pharmaceutical combination according to any one of embodiments 1-3, wherein a unit dose of the anti-TIM-3 antibody or the antigen-binding fragment thereof is 60-1800 mg, 100-1600 mg, 120-1600 mg, 180-1200 mg, or 240-600 mg; preferably, the unit dose of the anti-TIM-3 antibody or the antigen-binding fragment thereof is 240 mg, 300 mg, 360 mg, and/or 600 mg.
Embodiment 5: The pharmaceutical combination according to any one of embodiments 1-4, wherein a unit dose of the anti-PD-1 antibody or the antigen-binding fragment thereof is 10-500 mg, 50-500 mg, 50-200 mg, or 100-200 mg; preferably, the unit dose of the anti-PD-1 antibody or the antigen-binding fragment thereof is 100 mg and/or 200 mg.
Embodiment 6: The pharmaceutical combination according to any one of embodiments 1-5, wherein a mass ratio of the anti-TIM-3 antibody or the antigen-binding fragment thereof to the anti-PD-1 antibody or the antigen-binding fragment thereof is (0.1-180):1, (0.2-50):1, (0.2-9):1, (0.5-9):1, or (3-6):1.
Embodiment 7: The pharmaceutical combination according to any one of embodiments 1-6, wherein the pharmaceutical combination is suitable for administration within a single treatment cycle and comprises 100-1800 mg, 600-1800 mg, 600-1500 mg, or 600-1200 mg of the anti-TIM-3 antibody or the antigen-binding fragment thereof; preferably, the pharmaceutical combination is suitable for administration within a single treatment cycle and comprises 600 mg or 1200 mg of the anti-TIM-3 antibody or the antigen-binding fragment thereof.
Embodiment 8: The pharmaceutical combination according to embodiment 7, wherein the pharmaceutical combination further comprises 10-800 mg, 50-500 mg, or 100-200 mg of the anti-PD-1 antibody or the antigen-binding fragment thereof; preferably, the pharmaceutical combination further comprises 200 mg of the anti-PD-1 antibody or the antigen-binding fragment thereof.
Embodiment 9: The pharmaceutical combination according to any one of embodiments 1-8, wherein the anti-PD-1 antibody or the antigen-binding fragment thereof comprises: HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16.
Embodiment 10: The pharmaceutical combination according to any one of embodiments 1-9, wherein the anti-PD-1 antibody or the antigen-binding fragment thereof comprises: a heavy chain variable region having an amino acid sequence having at least 95% identity to the amino acid sequence set forth in SEQ ID NO: 17, and a light chain variable region having an amino acid sequence having at least 95% identity to the amino acid sequence set forth in SEQ ID NO: 18.
Embodiment 11: The pharmaceutical combination according to any one of embodiments 1-10, wherein the anti-PD-1 antibody or the antigen-binding fragment thereof comprises: a heavy chain having an amino acid sequence having at least 95% identity to the amino acid sequence set forth in SEQ ID NO: 19, and a light chain having an amino acid sequence having at least 95% identity to the amino acid sequence set forth in SEQ ID NO: 20.
Embodiment 12: The pharmaceutical combination according to any one of embodiments 1-11, wherein the anti-TIM-3 antibody or the antigen-binding fragment thereof and the anti-PD-1 antibody or the antigen-binding fragment thereof are formulated into a formulation for administration by a parenteral route; preferably, the anti-TIM-3 antibody or the antigen-binding fragment thereof and the anti-PD-1 antibody or the antigen-binding fragment thereof are formulated into a formulation for intravenous, intramuscular or subcutaneous administration.
Embodiment 13: The pharmaceutical combination according to any one of embodiments 1-12, wherein the anti-TIM-3 antibody or the antigen-binding fragment thereof and the anti-PD-1 antibody or the antigen-binding fragment thereof are formulated separately or in a single formulation.
Embodiment 14: The pharmaceutical combination according to any one of embodiments 1-13, wherein the pharmaceutical combination is for use in treating a tumor.
Embodiment 15: The pharmaceutical combination according to embodiment 14, wherein the tumor is a non-solid tumor.
Embodiment 16: The pharmaceutical combination according to embodiment 14, wherein the tumor is lymphoma.
Embodiment 17: The pharmaceutical combination according to embodiment 16, wherein the lymphoma is Hodgkin's lymphoma or non-Hodgkin's lymphoma.
Embodiment 18: Use of the pharmaceutical combination according to any one of embodiments 1-17 for preparing a medicament for use in treating a tumor.
Embodiment 19: The use according to embodiment 18, wherein the tumor is a non-solid tumor.
Embodiment 20: The use according to embodiment 18, wherein the tumor is lymphoma.
Embodiment 21: The use according to embodiment 20, wherein the lymphoma is Hodgkin's lymphoma or non-Hodgkin's lymphoma.
Embodiment 22: The use according to any one of embodiments 18-21, wherein the anti-TIM-3 antibody or the antigen-binding fragment thereof and the anti-PD-1 antibody or the antigen-binding fragment thereof can be administered simultaneously, sequentially, and/or alternately.
Embodiment 23: The use according to any one of embodiments 18-22, wherein the anti-TIM-3 antibody or the antigen-binding fragment thereof is administered once every 1 week, every 2 weeks, every 3 weeks, or every 4 weeks; preferably, the anti-TIM-3 antibody or the antigen-binding fragment thereof is administered once every 3 weeks.
Embodiment 24: The use according to any one of embodiments 18-23, wherein the anti-TIM-3 antibody or the antigen-binding fragment thereof is administered at a dose of 100-1800 mg, 600-1800 mg, 600-1500 mg, or 600-1200 mg of the anti-TIM-3 antibody or the antigen-binding fragment thereof each time; preferably, the anti-TIM-3 antibody or the antigen-binding fragment thereof is administered at a dose of 600 mg or 1200 mg of the anti-TIM-3 antibody or the antigen-binding fragment thereof each time.
Embodiment 25: The use according to any one of embodiments 18-24, wherein the anti-PD-1 antibody or the antigen-binding fragment thereof is administered once every 1 week, every 2 weeks, every 3 weeks, or every 4 weeks; preferably, the anti-PD-1 antibody or the antigen-binding fragment thereof is administered once every 3 weeks.
Embodiment 26: The use according to any one of embodiments 18-25, wherein the anti-PD-1 antibody or the antigen-binding fragment thereof is administered at a dose of 10-800 mg, 50-500 mg, or 100-200 mg of the anti-PD-1 antibody or the antigen-binding fragment thereof each time; preferably, the anti-PD-1 antibody or the antigen-binding fragment thereof is administered at a dose of 200 mg of the anti-PD-1 antibody or the antigen-binding fragment thereof each time.
Embodiment 27: A kit for use in treating a tumor, comprising the pharmaceutical combination according to any one of embodiments 1-17.
Embodiment 28: The kit according to embodiment 27, wherein the tumor is a non-solid tumor.
Embodiment 29: The kit according to embodiment 27, wherein the tumor is lymphoma.
Embodiment 30: The kit according to embodiment 29, wherein the lymphoma is Hodgkin's lymphoma or non-Hodgkin's lymphoma.

### Technical effects

Generally, use of the above pharmaceutical combination of the present disclosure will provide:
(1) better efficacy in reducing the growth of tumors or even eliminating the tumors as compared with either drug of the combination administered alone;
(2) fewer doses as compared with either drug of the combination administered alone;
(3) treatment that is well tolerated in patients with fewer adverse effects and/or complications as compared with either drug administered alone;
(4) a higher disease control rate in patients treated;
(5) a prolonged survival (e.g., median survival time, progression-free survival, or overall survival) in patients treated;
(6) a prolonged survival (e.g., median survival time, progression-free survival, or overall survival) or tolerability in patients treated as compared with standard chemotherapies;
(7) a prolonged duration of response (DOR); and/or
(8) good anti-tumor activity and better synergistic anti-tumor effect, as compared with either drug of the combination administered alone.

The pharmaceutical combination and treatment of the present disclosure have relatively good efficacy in treating lymphoma, particularly recurrent or refractory lymphoma (e.g., recurrent or refractory Hodgkin's lymphoma, recurrent or refractory diffuse large B-cell lymphoma, recurrent or refractory follicular lymphoma, and recurrent or refractory peripheral T-cell lymphoma), with beneficial effects in at least one of ORR, DCR, DOR, PFS, OS, tolerability, and side effects.

### Definitions and explanations

Unless otherwise stated, the following terms used in the present disclosure shall have the following meanings. A certain term, unless otherwise specifically defined, should not be considered uncertain or unclear, but construed according to its common meaning in the art. When referring to a trade name in the present disclosure, it is intended to refer to its corresponding commercial product or its active ingredient.

As used herein, the term "pharmaceutical combination" refers to a combination of two or more active ingredients (including administration in the form of the respective active ingredients themselves, or in the form of their respective derivatives (such as pharmaceutically acceptable salts or esters), prodrugs, or compositions) that are administered simultaneously or sequentially. The active ingredients can be administered to a subject simultaneously or sequentially in any order as individual formulations. Alternatively, all of the active ingredients are formulated in a single formulation for simultaneous administration to a subject.

The term "fixed combination" refers to simultaneous administration of the active components (e.g., the anti-TIM-3 antibody or the antigen-binding fragment thereof and the anti-PD-1 antibody or the antigen-binding fragment thereof) to a subject at a fixed total dose or in a fixed dose proportion, or in the form of a single substance, pharmaceutical composition or formulation.

The term "non-fixed combination" refers to simultaneous, parallel, or sequential administration of two or more aforementioned active components as independent substances (e.g., pharmaceutical compositions and formulations) to a subject, wherein the active components administered to the subject reach therapeutically effective amounts. An enumerable example of the non-fixed combination is cocktail therapy, for example, 3 or more active components are administered. In a non-fixed combination, each of the active components can be packaged, sold, or administered as a fully independent pharmaceutical composition. The "non-fixed combination" also includes the combined use of "fixed combinations", or a "fixed combination" and an independent substance of any one or more active components.

As used herein, the term "antibody" refers to an antigen-binding protein having at least one antigen-binding domain. The antibody and the fragment thereof of the present disclosure may be an intact antibody or any fragment thereof. Thus, the antibody and the fragment thereof of the present disclosure include a monoclonal antibody or a fragment thereof and an antibody variant or a fragment thereof. Examples of the antibody and the antigen-binding fragment thereof include a monospecific antibody, a bispecific antibody, a multispecific antibody, a Fab fragment, a Fab' fragment, an F(ab)'₂ fragment, an Fv fragment, an isolated CDR, a single-chain Fv (scFv) molecule, and other antibody fragments known in the art. The anti-TIM-3 antibody, the anti-PD-1 antibody, and the antigen-binding fragments thereof disclosed herein may be of the IgG1, IgG2, IgG3, or IgG4 isotype. The term "isotype" refers to the class of antibodies encoded by the heavy chain constant region gene. The anti-TIM-3 antibody and the antigen-binding fragment thereof and the anti-PD-1 antibody and the antigen-binding fragment thereof of the present disclosure may be derived from any species, including, but not limited to, mouse, rat, rabbit, primate, llama, and human. The anti-TIM-3 antibody and the antigen-binding fragment thereof and the anti-PD-1 antibody and the antigen-binding fragment thereof of the present disclosure may be murine antibodies, chimeric antibodies, humanized antibodies, or fully human-derived antibodies. Unless otherwise stated, the "antibody" in the present disclosure includes an intact antibody and any antigen-binding fragment (i.e., "antigen-binding portion") or single chain thereof. A conventional "intact antibody" is a glycoprotein comprising two heavy (H) chains and two light (L) chains, where the heavy chains and the light chains are linked by disulfide bonds. Each heavy chain consists of a heavy chain variable region (VH) and a heavy chain constant region (CH). The heavy chain constant region consists of three domains: CH1, CH2, and CH3. Each light chain consists of a light chain variable region (VL) and a light chain constant region (CL). The light chain constant region consists of one domain CL. The VH and VL regions can further be divided into hypervariable regions, i.e., complementarity determining regions (CDRs), and framework regions (FRs) with relatively conservative sequences. Each VH and VL consists of three CDRs and four FRs arranged from the amino-terminus to the carboxyl-terminus as follows: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component of the classical complement system (C1q). Meanwhile, as known to those skilled in the art, a special "intact antibody", such as a nanobody, has only heavy (H) chains and lacks light (L) chains.

The "antigen-binding fragment" or "antibody-binding portion" of the antibody refers to one or more fragments of the antibody that retain the function of specifically binding to antigen (e.g., TIM-3 or PD-1). It has been demonstrated that the antigen-binding function of an antibody can be implemented by fragments of the intact antibody. Examples encompassed within the term "antigen-binding portion/fragment" of an antibody include: (i) a Fab fragment: a monovalent fragment consisting of V_{L}, V_{H}, CL and CH1 domains; (ii) an F(ab')2 fragment, a bivalent fragment comprising two Fab fragments linked by a disulfide bridge in the hinge region; (iii) an Fd fragment consisting of VH and CH1 domains; (iv) an Fv fragment consisting of VL and VH domains of a single arm of the antibody; (v) a dAb fragment consisting of a VH domain (see Ward et al., Nature. 341:544-546 (1989)); (vi) an isolated complementarity determining region (CDR); and (vii) a nanobody, a heavy chain variable region comprising a single variable domain and two constant domains. Furthermore, although the two domains VL and VH of the Fv fragment are encoded by different genes, the VL and VH domains can be joined via a synthetic linker by recombinant means into a single protein chain in which VL pairs with VH to form a monovalent molecule (referred to as a single-chain Fv (scFv); see, e.g., Bird et al., Science. 242:423-426 (1988); Huston et al., Proc. Natl. Acad. Sci. 85:5879-5883 (1988)). These single-chain antibodies are also encompassed within the term antigen-binding portion/fragment. Furthermore, a recombinant polypeptide, a fusion protein, and an immunoconjugate comprising the antigen-binding portion/fragment are also encompassed within the term antigen-binding portion/fragment.

The "chimeric antibody" refers to an antibody having at least a portion of the heavy chain variable region and a portion of the light chain variable region derived from one species, and at least a portion of the constant region derived from another species. For example, in one embodiment, the chimeric antibody may comprise a murine variable region and a human constant region.

The "humanized antibody" refers to an antibody comprising complementarity determining regions (CDRs) derived from a non-human antibody, and framework and constant regions derived from a human antibody. For example, the anti-TIM-3 antibody and the anti-PD-1 antibody may comprise CDRs derived from one or more murine antibodies and human framework and constant regions. Exemplary humanized antibodies are provided herein. Additional anti-TIM-3 antibodies or variants thereof comprising the HCDRs and LCDRs provided herein can be produced using any human framework sequences, and are also included in the present disclosure. Additional anti-PD-1 antibodies or variants thereof comprising the HCDRs and LCDRs provided herein can be produced using any human framework sequences, and are also included in the present disclosure. In one embodiment, framework sequences suitable for use in the present disclosure include those similar in structure to the framework sequences provided herein. Additional modifications may be made in the framework regions to improve the properties of the antibodies provided herein. Such additional framework modifications may include chemical modifications, point mutations for reducing immunogenicity or removing T cell epitopes, or modifications reverting the mutations to residues in original gennline sequences. In some embodiments, such modifications include those corresponding to the mutations exemplified herein, including reverse mutations to germline sequences. For example, in one embodiment, one or more amino acids in the human VH and/or VL framework regions of the humanized antibodies disclosed herein are reverted to the corresponding amino acids in the parent murine antibodies.

The term "identity" is also known as homology. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

The term "treat", "treating", or "treatment" refers to the attempt to alter the natural course of a disease in a treated individual and can be for the purpose of preventing, ameliorating, or eliminating the disease or one or more symptoms associated with the disease, including, but not limited to, preventing the occurrence or recurrence of the disease, alleviating the symptoms of the disease, reducing any direct or indirect pathological consequences of the disease, preventing the metastasis of the disease, slowing the progression rate of the disease, ameliorating or alleviating the state of the disease, extending the frequency and duration of the asymptomatic phase, and regressing or improving the prognosis of the disease.

The term "therapeutically effective amount" refers to an amount of the compound of the present disclosure for (i) treating a specific disease, condition, or disorder, (ii) alleviating, ameliorating, or eliminating one or more symptoms of the specific disease, condition, or disorder, or (iii) preventing or delaying the onset of one or more symptoms of the specific disease, condition, or disorder described herein. The amount of active substance (e.g., the antibody or the antigen-binding fragment thereof of the present disclosure) constituting the "therapeutically effective amount" may vary according to factors such as the disease state, age, sex, and weight of the individual, and the ability of a therapeutic agent or a combination of therapeutic agents to elicit a desired response in the individual. The therapeutically effective amount may also be determined routinely by those skilled in the art following their knowledge and the present disclosure.

The term "administer", "administration", or "administering" refers to physically introducing a therapeutic agent to an entity using any of a variety of methods and delivery systems known to those skilled in the art.

Routes of administration of an antibody or an antigen-binding fragment thereof (e.g., the anti-TIM-3 antibody or the antigen-binding fragment thereof or the anti-PD-1 antibody or the antigen-binding fragment thereof) include intravenous, intramuscular, subcutaneous, intraperitoneal, spinal, or other parenteral routes of administration, e.g., by injection or infusion. As used herein, the phrase "parenteral administration" refers to modes of administration apart from gastrointestinal administration, typically by injection, including, but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and in vivo electroporation. The administration may also be performed, for example, once, multiple times, and/or over one or more extended periods of time.

The term "flat dose" refers to a dose administered to a patient without considering the weight or the body surface area (BSA) of the patient. Thus, the flat dose is specified as the absolute amount of a medicament rather than the mg/kg dose. For example, a 60 kg human and a 100 kg human will receive the same dose of an antibody (e.g., 200 mg of the anti-PD-1 antibody or the antigen-binding fragment thereof).

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

Herein, the terms "subject", "patient", and "entity" are used interchangeably. The "subject", "patient", or "entity" includes any human or non-human animal. The term "non-human animal" includes, but is not limited to, vertebrates such as non-human primates, sheep, dogs, and rodents such as mice, rats, and guinea pigs. In some embodiments, the term "subject", "patient", or "entity" refers to a mammal. In some embodiments, the subject, patient, or entity is a mouse. In some embodiments, the subject, patient, or entity is a human.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to ±5%, for example, fluctuating within a particular numerical range given ±2%, ±1%, or ±0.5%. Where a particular value is given in the present disclosure or in the claims, unless otherwise stated, "about" should be considered to mean being within an acceptable error range for that particular value. Herein, unless otherwise stated, the values for the dose, timing, step parameters, or conditions of the drug in the present disclosure are all modified by "about" by default.

As used herein, "combined use" or "use in combination" means that two or more active substances may be administered to a subject simultaneously or sequentially in any order as individual formulations. Alternatively, all of the active ingredients are formulated in a single formulation for simultaneous administration to a subject.

The term "single dose" refers to the smallest unit of packaging containing a certain amount of a pharmaceutical product; for example, in a box of seven tablets, one tablet is a single dose; or a vial of injection is a single dose. The term "multiple doses" consists of multiple single doses. "Unit dose" refers to a dose of an active ingredient contained in a minimum packaging unit containing a certain amount of a pharmaceutical product, for example, a dose of an antibody contained in a vial of an antibody injection solution is a unit dose.

The term "pharmaceutical composition" refers to a mixture consisting of one or more of the active ingredients or the pharmaceutical combinations thereof of the present disclosure and a pharmaceutically acceptable excipient. The pharmaceutical composition is intended to facilitate the administration of the compound or the pharmaceutical combination thereof of the present disclosure to a subject. Herein, the terms "pharmaceutical composition" and "formulation" have the same meaning and are used interchangeably.

The term "recurrent" cancer is one that regenerates at an initial site or a distant site after being responsive to initial treatment (e.g., surgery). A "locally recurrent" cancer is one that occurs, after treatment, at the same location as the previously treated cancer.

As used herein, "treatment failure" is defined as the occurrence of disease progression or recurrence during the treatment or after the last treatment.

The term "resistant" means that administration of a therapeutic agent at a normal rate of administration or at a frequency tolerated by a patient has no substantial effect on the cancer.

The term "systemic" treatment refers to treatment in which a drug travels through the bloodstream to reach and affect cells throughout the body.

Herein, unless otherwise specified clearly, singular terms encompass plural referents, and vice versa.

All patents, patent applications and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

### Examples

For clarity, the present disclosure is further described with the following examples, which are, however, not intended to limit the scope of the present disclosure. All the reagents used in the present disclosure are commercially available and can be used without further purification.

The content of the patent application WO2020041520 or CN112566936 is incorporated herein in its entirety. In the following examples, the anti-TIM-3 antibody comprises a heavy chain having the amino acid sequence set forth in SEQ ID NO: 9 of the present disclosure, and a light chain having the amino acid sequence set forth in SEQ ID NO: 10 of the present disclosure.

The content of the patent application CN106977602A is incorporated herein in its entirety. In the following examples, the 14C12H1L1 is 14C12H1L1 in CN106977602A, and the 14C12H1L1 comprises a heavy chain having the amino acid sequence set forth in SEQ ID NO: 19 of the present disclosure, and a light chain having the amino acid sequence set forth in SEQ ID NO: 20 of the present disclosure.

### Example 1: Clinical Trial for Recurrent or Refractory Lymphoma

### 1. Inclusion criteria

Subjects who met all of the following inclusion criteria could be enrolled in this trial:
(1) Voluntary participation and signedinformed consent;
(2) No gender limitation, aged 18-75 years; ECOG (PS) score: 0-1; expected survival time ≥ 3 months;
(3) Pathologically or cytologically diagnosed recurrent or refractory lymphoma, with disease progression during or after the most recent treatment, or without objective response confirmed after adequate treatment; while satisfying one of the following criteria:
   a. Classical Hodgkin's lymphoma (cHL): has previously received at least a second-line systemic treatment and is resistant to PD-1 or PD-L1;
   b. B-cell non-Hodgkin's lymphoma (B-NHL): has previously received at least a second-line systemic treatment and at least one therapy containing anti-CD20 targeted treatment (for indolent B-cell non-Hodgkin's lymphoma, it also needs to meet at least one treatment indication as specified by the guidelines for this type of lymphoma);
   c. T-cell non-Hodgkin's lymphoma (T-NHL): has previously received at least a first-line systemic treatment ;
(4) At least one radiologically measurable tumor lesion (the long diameter of a nodal lesion > 15 mm, and the long diameter of an extranodal lesion > 10 mm, based on the Lugano evaluation criteria, 2014 edition) present in 2 vertical directions, as evaluated by CT or MRI;
(5) Main organ functions in the screening period meet the following criteria:
   a. Criteria for blood routine examination (no growth factor used or no blood transfusion conducted within 7 days prior to the examination):
      Absolute neutrophil count (ANC) ≥ 1.5 × 10⁹/L; absolute lymphocyte count (ALC) ≥ 0.5 × 10⁹/L; CD4+ T lymphocyte count ≥ 0.2 × 10⁹/L; hemoglobin (Hb) ≥ 80 g/L; platelet (PLT) ≥ 75 × 10⁹/L;
   b. Criteria for blood biochemical examination:
      Alanine transaminase (ALT) and aspartate transaminase (AST) ≤ 2.5 × upper limit of normal (ULN) (for those with liver involvement of lymphoma or biliary obstruction, ≤ 5 × ULN); serum total bilirubin (TBIL) ≤ 1.5 × ULN; blood coagulation function: activated partial thromboplastin time (APTT), international normalized ratio (INR), prothrombin time (PT) ≤ 1.5 × ULN; serum creatinine (Cr) ≤ 1.5 × ULN or creatinine clearance rate ≥ 50 mL/min; thyroid function: thyroid-stimulating hormone (TSH) ≤ ULN; if there are abnormalities, serum free triiodothyronine (FT3) and free tetraiodothyronine (FT4) should be examined, and if FT3 and FT4 levels are normal, the subject can be enrolled;
   c. Heart color ultrasound: left ventricular ejection fraction (LVEF) ≥ 50%;
(6) Female subjects of childbearing age should agree to take contraceptive measures (intrauterine devices [IUD], contraceptives, or condoms) during the study and for 6 months after the study; serum or urine pregnancy test results should be negative within 7 days before enrollment, and the subjects must not be breastfeeding; male subjects should agree to take contraceptive measures during the study and for 6 months after the study.

### 2. Test drugs and administration regimens

Infusion of a 14C12H1L1 injection was performed first, followed by infusion of an anti-TIM-3 antibody injection. 14C12H1L1 injection (strength: 100 mg/10 mL/vial): with every 3 weeks counted as 1 treatment cycle, administered by intravenous infusion once on day 1 of each treatment cycle at a dose of 200 mg of 14C12H1L1.

Anti-TIM-3 antibody injection (strength: 240 mg/4 mL/vial, or 600 mg/10 mL/vial): with every 3 weeks counted as 1 treatment cycle, administered by intravenous infusion once on day 1 of each treatment cycle at a dose of 600 mg or 1200 mg of anti-TIM-3 antibody.

### 3. Evaluation criteria

The evaluation of efficacy was based on the Lugano evaluation criteria, 2014 edition, and the efficacy was confirmed using the LYRIC 2016 criteria.

The severity of adverse events was determined using the NCI-CTC AE 5.0 criteria.

### 4. Endpoints

Objective response rate (ORR), complete response rate (CRR), disease control rate (DCR), duration of response (DOR), progression-free survival (PFS), overall survival (OS), and the like; pharmacokinetic (PK) parameters; receptor occupancy (RO) status; immunogenicity-related indicators: incidence and titers of anti-drug antibodies (ADAs), incidence of neutralizing antibodies (Nabs); incidence of adverse events: occurrence of all adverse events (AEs), severe adverse events (SAEs), and treatment-related adverse events (TRAEs), and abnormal laboratory examination indicators.

### 5. Results

### Baseline characteristics

A total of 10 patients were recruited, including 6 males and 4 females. Among them, 3 patients had classical Hodgkin's lymphoma, 6 patients had diffuse large B-cell lymphoma, and 1 patient had peripheral T-cell lymphoma.

### Safety and effectiveness

The patients exhibited good tolerance to the combination of the anti-TIM-3 antibody with 14C12H1L1 of the present disclosure, with most experiencing Grade 1 or 2 AEs. Based on the PET-CT results, as evaluated based on the Lugano evaluation criteria, 2014 edition, an objective response was observed in 4 out of 8 evaluable patients. Among those with classical Hodgkin's lymphoma, 2 out of 3 evaluable patients showed a partial response (PR), while 1 evaluable patient exhibited stable disease (SD), resulting in a disease control rate (DCR) of 100%. Among those with diffuse large B-cell lymphoma, 1 out of 4 evaluable patients showed a partial response (PR), while 1 evaluable patient exhibited stable disease (SD). Among the patients with peripheral T-cell lymphoma, 1 out of 1 evaluable patient showed a partial response (PR). The combination of the anti-TIM-3 antibody with 14C12H1L1 of the present disclosure could safely and effectively treat lymphoma, particularly recurrent or refractory lymphoma.

**Table 3. Details of exemplary patients**

| Classical Hodgkin's lymphoma | |
|---|---|
| 001 | Diagnostic result: classical Hodgkin's lymphoma (Lugano stage II) |
| | Previous treatment history: ABVD chemotherapy (epirubicin + bleomycin + vindesine sulfate + imidazole carboxamide), DHAP chemotherapy (dexamethasone + cytarabine + cisplatin), GLS-010 (anti-PD-1 antibody) injection, F0002-ADC (anti-CD30-MCC-DM1), PM8001 (PD-1/TGF-β bispecific fusion protein) injection, and radiation therapy |
| | Treatment regimen: 200 mg of 14C12H1L1 + 600 mg of anti-TIM-3 antibody per treatment cycle |
| | Treatment outcome: the patient was evaluated as having PR at week 6 and remained PR at week 36; the patient continued to receive treatment in the group |
| 002 | Diagnostic result: classical Hodgkin's lymphoma (Lugano stage IV) |
| | Previous treatment history: ABVD chemotherapy (dacarbazine + liposomal adriamycin + vincristine sulfate + bleomycin hydrochloride, or dacarbazine + pirarubicin hydrochloride + vincristine sulfate + bleomycin hydrochloride), and sintilimab injection |
| | Treatment regimen: 200 mg of 14C12H1L1 + 1200 mg of anti-TIM-3 antibody per treatment cycle |
| | Treatment outcome: the patient was evaluated as having PR |
| 003 | Diagnostic result: classical Hodgkin's lymphoma (Lugano stage IV) |
| | Previous treatment history: ABVD chemotherapy, DHAP chemotherapy, anti-PD-1 antibody + CHOP chemotherapy (cyclophosphamide + doxorubicin + vincristine + prednisone acetate), and SYHX1903 (protein kinase 9 inhibitor) |
| | Treatment regimen: 200 mg of 14C12H1L1 + 1200 mg of anti-TIM-3 antibody per treatment cycle |
| | Treatment outcome: the patient was evaluated as having SD, with a Deauville score of 2; the patient continued to receive treatment in the group |

| Diffuse large B-cell lymphoma | |
|---|---|
| 004 | Diagnostic result: diffuse large B-cell lymphoma (Lugano stage IV) |
| | Previous treatment history: RI-CHOP chemotherapy (rituximab + cyclophosphamide + pirarubicin hydrochloride + vincristine sulfate + prednisone acetate), R2-ICE chemotherapy (rituximab + mesna + lenalidomide + ifosfamide + carboplatin + etoposide), and SYHX1903 (CDK9 inhibitor) |
| | Treatment regimen: 200 mg of 14C12H1L1 + 600 mg of anti-TIM-3 antibody per treatment cycle |
| | Treatment outcome: the patient was evaluated as having PR at week 6 and remained PR at week 42; the patient continued to receive treatment in the group |
| 005 | Diagnostic result: diffuse large B-cell lymphoma (Lugano stage IV) |
| | Previous treatment history: R-CHOP-MTX chemotherapy (rituximab + cyclophosphamide + doxorubicin hydrochloride liposome + vincristine sulfate + prednisone acetate + methotrexate) and lenalidomide |
| | Treatment regimen: 200 mg of 14C12H1L1 + 1200 mg of anti-TIM-3 antibody per treatment cycle |
| | Treatment outcome: the patient was evaluated as having SD |

| Peripheral T-cell lymphoma | |
|---|---|
| 006 | Diagnostic result: peripheral T-cell lymphoma (Lugano stage IV) |
| | Previous treatment history: CDOP chemotherapy (cyclophosphamide + doxorubicin hydrochloride liposome + vincristine sulfate + prednisone) + DEP chemotherapy regimen (liposomal adriamycin + etoposide + dexamethasone), GEMOX chemotherapy regimen (gemcitabine + oxaliplatin) + ATG-010, anti-PD-1 antibody + chidamide, zimberelimab + bleomycin hydrochloride + paclitaxel, doxorubicin hydrochloride liposome + gemcitabine + cotuximab, azacitidine + etoposide + paclitaxel, bleomycin hydrochloride + gemcitabine hydrochloride + thalidomide + selinexor, and topotecan hydrochloride + bleomycin hydrochloride + anti-PD-L1 antibody |
| | Treatment regimen: 200 mg of 14C12H1L1 + 600 mg of anti-TIM-3 antibody per treatment cycle |
| | Treatment outcome: the patient was evaluated as having PR |

## Claims

1. A pharmaceutical combination, comprising an anti-TIM-3 antibody or an antigen-binding fragment thereof and an anti-PD-1 antibody or an antigen-binding fragment thereof, wherein the anti-TIM-3 antibody or the antigen-binding fragment thereof comprises:
(1) HCDR1 of the amino acid sequence set forth in SEQ ID NO: 1, HCDR2 of the amino acid sequence set forth in SEQ ID NO: 2, HCDR3 of the amino acid sequence set forth in SEQ ID NO: 3, LCDR1 of the amino acid sequence set forth in SEQ ID NO: 4, LCDR2 of the amino acid sequence set forth in SEQ ID NO: 5, and LCDR3 of the amino acid sequence set forth in SEQ ID NO: 6;
(2) HCDR1 of the amino acid sequence set forth in SEQ ID NO: 21, HCDR2 of the amino acid sequence set forth in SEQ ID NO: 22, HCDR3 of the amino acid sequence set forth in SEQ ID NO: 23, LCDR1 of the amino acid sequence set forth in SEQ ID NO: 24, LCDR2 of the amino acid sequence set forth in SEQ ID NO: 25, and LCDR3 of the amino acid sequence set forth in SEQ ID NO: 26;
(3) a heavy chain variable region having an amino acid sequence having at least 95% identity to the amino acid sequence set forth in SEQ ID NO: 7, and a light chain variable region having an amino acid sequence having at least 95% identity to the amino acid sequence set forth in SEQ ID NO: 8;
(4) a heavy chain variable region having an amino acid sequence having at least 95% identity to the amino acid sequence set forth in SEQ ID NO: 27, and a light chain variable region having an amino acid sequence having at least 95% identity to the amino acid sequence set forth in SEQ ID NO: 28;
(5) a heavy chain having an amino acid sequence having at least 95% identity to the amino acid sequence set forth in SEQ ID NO: 9, and a light chain having an amino acid sequence having at least 95% identity to the amino acid sequence set forth in SEQ ID NO: 10; or
(6) a heavy chain having an amino acid sequence having at least 95% identity to the amino acid sequence set forth in SEQ ID NO: 29, and a light chain having an amino acid sequence having at least 95% identity to the amino acid sequence set forth in SEQ ID NO: 30.

2. The pharmaceutical combination according to claim 1, wherein a unit dose of the anti-TIM-3 antibody or the antigen-binding fragment thereof is 60-1800 mg, 100-1600 mg, 120-1600 mg, 180-1200 mg, or 240-600 mg; preferably, the unit dose of the anti-TIM-3 antibody or the antigen-binding fragment thereof is 240 mg, 300 mg, 360 mg, and/or 600 mg.

3. The pharmaceutical combination according to claim 1 or 2, wherein a unit dose of the anti-PD-1 antibody or the antigen-binding fragment thereof is 10-500 mg, 50-500 mg, 50-200 mg, or 100-200 mg; preferably, the unit dose of the anti-PD-1 antibody or the antigen-binding fragment thereof is 100 mg and/or 200 mg.

4. The pharmaceutical combination according to any one of claims 1-3, wherein a mass ratio of the anti-TIM-3 antibody or the antigen-binding fragment thereof to the anti-PD-1 antibody or the antigen-binding fragment thereof is (0.1-180):1, (0.2-50):1, (0.2-9):1, (0.5-9):1, or (3-6):1.

5. The pharmaceutical combination according to any one of claims 1-4, wherein the pharmaceutical combination is suitable for administration within a single treatment cycle and comprises 100-1800 mg, 600-1800 mg, 600-1500 mg, or 600-1200 mg of the anti-TIM-3 antibody or the antigen-binding fragment thereof; preferably, the pharmaceutical combination is suitable for administration within a single treatment cycle and comprises 600 mg or 1200 mg of the anti-TIM-3 antibody or the antigen-binding fragment thereof; optionally, the pharmaceutical combination further comprises 10-800 mg, 50-500 mg, or 100-200 mg of the anti-PD-1 antibody or the antigen-binding fragment thereof; preferably, the pharmaceutical combination further comprises 200 mg of the anti-PD-1 antibody or the antigen-binding fragment thereof.

6. The pharmaceutical combination according to any one of claims 1-5, wherein the anti-PD-1 antibody or the antigen-binding fragment thereof comprises:
(1) HCDR1 of the amino acid sequence set forth in SEQ ID NO: 11, HCDR2 of the amino acid sequence set forth in SEQ ID NO: 12, HCDR3 of the amino acid sequence set forth in SEQ ID NO: 13, LCDR1 of the amino acid sequence set forth in SEQ ID NO: 14, LCDR2 of the amino acid sequence set forth in SEQ ID NO: 15, and LCDR3 of the amino acid sequence set forth in SEQ ID NO: 16;
(2) a heavy chain variable region having an amino acid sequence having at least 95% identity to the amino acid sequence set forth in SEQ ID NO: 17, and a light chain variable region having an amino acid sequence having at least 95% identity to the amino acid sequence set forth in SEQ ID NO: 18; or
(3) a heavy chain having an amino acid sequence having at least 95% identity to the amino acid sequence set forth in SEQ ID NO: 19, and a light chain having an amino acid sequence having at least 95% identity to the amino acid sequence set forth in SEQ ID NO: 20.

7. The pharmaceutical combination according to any one of claims 1-6, wherein the anti-TIM-3 antibody or the antigen-binding fragment thereof and the anti-PD-1 antibody or the antigen-binding fragment thereof are formulated into a formulation for administration by a parenteral route; preferably, the anti-TIM-3 antibody or the antigen-binding fragment thereof and the anti-PD-1 antibody or the antigen-binding fragment thereof are formulated into a formulation for intravenous, intramuscular or subcutaneous administration.

8. The pharmaceutical combination according to any one of claims 1-7, wherein the anti-TIM-3 antibody or the antigen-binding fragment thereof and the anti-PD-1 antibody or the antigen-binding fragment thereof are formulated separately or in a single formulation.

9. The pharmaceutical combination according to any one of claims 1-8, wherein the pharmaceutical combination is for use in treating a tumor; preferably, the tumor is a non-solid tumor or lymphoma; and more preferably, the lymphoma is Hodgkin's lymphoma or non-Hodgkin's lymphoma.

10. Use of the pharmaceutical combination according to any one of claims 1-8 for preparing a medicament for use in treating a tumor, wherein preferably, the tumor is a non-solid tumor or lymphoma; and more preferably, the lymphoma is Hodgkin's lymphoma or non-Hodgkin's lymphoma.

11. The use according to claim 10, wherein the anti-TIM-3 antibody or the antigen-binding fragment thereof and the anti-PD-1 antibody or the antigen-binding fragment thereof can be administered simultaneously, sequentially, and/or alternately.

12. The use according to claim 10 or 11, wherein the anti-TIM-3 antibody or the antigen-binding fragment thereof is administered once every 1 week, every 2 weeks, every 3 weeks, or every 4 weeks; preferably, the anti-TIM-3 antibody or the antigen-binding fragment thereof is administered once every 3 weeks;
optionally, the anti-PD-1 antibody or the antigen-binding fragment thereof is administered once every 1 week, every 2 weeks, every 3 weeks, or every 4 weeks; preferably, the anti-PD-1 antibody or the antigen-binding fragment thereof is administered once every 3 weeks.

13. The use according to any one of claims 10-12, wherein the anti-TIM-3 antibody or the antigen-binding fragment thereof is administered at a dose of 100-1800 mg, 600-1800 mg, 600-1500 mg, or 600-1200 mg of the anti-TIM-3 antibody or the antigen-binding fragment thereof each time; preferably, the anti-TIM-3 antibody or the antigen-binding fragment thereof is administered at a dose of 600 mg or 1200 mg of the anti-TIM-3 antibody or the antigen-binding fragment thereof each time.

14. The use according to any one of claims 10-13, wherein the anti-PD-1 antibody or the antigen-binding fragment thereof is administered at a dose of 10-800 mg, 50-500 mg, or 100-200 mg of the anti-PD-1 antibody or the antigen-binding fragment thereof each time; preferably, the anti-PD-1 antibody or the antigen-binding fragment thereof is administered at a dose of 200 mg of the anti-PD-1 antibody or the antigen-binding fragment thereof each time.

15. A kit for use in treating a tumor, comprising the pharmaceutical combination according to any one of claims 1-8.
